# FASCICULE DE BREVET EUROPEEN

(11) **EP 1 371 658 B1**
(45) Date de publication et mention de la délivrance du brevet: **12.09.2007**
(21) Numéro de dépôt: 03291142.2
(22) Date de dépôt: 15.05.2003
(51) Int. Cl.: C07H 13/06, C07H 1/00

(54) **Dérivé du glucose et de vitamine F, compositions le comprenant, utilisations et procédé de préparation**
Glucose und Vitamin F Derivate, Zusammensetzungen, Verwendung und Verfahren zu deren Herstellung
Derivative of glucose and vitamin F, compositions containing it, its uses and its preparation

(30) Priorité: 13.06.2002 FR 0207293; 13.06.2002 FR 0207290
(43) Date de publication de la demande: 17.12.2003
(60) Demande divisionnaire: 07110772.6
(73) Titulaire: L'ORÉAL, 75008 Paris (FR)
(72) Inventeur: Michelet, Jean-François, 94000 Creteil (FR); Dalko, Maria, 91190 GIF S/YVETTE (FR); Bernard, Bruno, 92200 Neuilly sur Seine (FR); Semeria, Didier, 93190 Livry-Gargan (FR); Philippe, Michel, 91320 Wissous (FR)
(74) Mandataire: Dodin, Catherine

(56) Documents cités:
- WO-A-93/02657
- US-A- 4 696 916
- US-A- 5 268 180

## Description

La présente invention a trait à un nouveau dérivé O-acylé du glucose, à son utilisation notamment en cosmétique ou en pharmacie en particulier pour lutter contre la chute des cheveux, ainsi que les compositions notamment cosmétiques ou pharmaceutiques le comprenant. L'invention concerne également un nouveau procédé de préparation de dérivés du glucose, lesdits dérivés étant O-acylés majoritairement en position 6 du glucose.

Chez l'être humain, la croissance des cheveux et leur renouvellement sont principalement déterminés par l'activité des follicules pileux et de leur environnement dermo-épidermique. Leur activité est cyclique et comporte essentiellement trois phases, à savoir la phase anagène, la phase catagène et la phase télogène.
A la phase anagène active ou phase de croissance, qui dure plusieurs années et au cours de laquelle les cheveux s'allongent, succède une phase catagène très courte et transitoire qui dure quelques semaines, puis une phase de repos, appelée phase télogène, qui dure quelques mois.
A la fin de la période de repos, les cheveux tombent et un autre cycle recommence. La chevelure se renouvelle donc en permanence, et sur les 150000 cheveux environ que comporte une chevelure, à chaque instant, 10% d'entre eux environ sont au repos et seront donc remplacés en quelques mois.

Dans un nombre important de cas, la chute précoce des cheveux survient chez des sujets prédisposés génétiquement et elle atteint notamment les hommes. Il s'agit plus particulièrement de l'alopécie androgénétique ou androgénique ou encore androgéno-génétique.
Cette alopécie est essentiellement due à une perturbation du renouvellement capillaire qui entraîne, dans un premier temps, l'accélération de la fréquence des cycles aux dépens de la qualité des cheveux puis de leur quantité. Il se produit un appauvrissement progressif de la chevelure par régression des cheveux dits "terminaux" au stade de duvets. Des zones sont touchées préférentiellement; notamment chez l'homme, les golfes temporaux ou frontaux ainsi que la partie supérieure de l'occipital, tandis que chez les femmes on constate plutôt une alopécie diffuse du vertex.

On recherche depuis de nombreuses années, notamment dans l'industrie cosmétique, des substances permettant de supprimer ou de réduire l'effet de l'alopécie, et notamment d'induire ou de stimuler la croissance des cheveux et/ou des poils, voire de diminuer ou retarder leur chute.
Dans cette optique, on a déjà proposé un grand nombre de composés actifs très divers, comme par exemple le 2,4-diamino 6-piperidino pyrimidine 3-oxyde ou "Minoxidil", ainsi que ses nombreux dérivés.
On a également proposé, notamment dans EP211610, des oligosaccharides contenant au moins une unité disaccharide constituée d'un reste d'acide uronique et d'un reste d'hexosamine.

La demande WO-A-93/02657 quant à elle enseigne que des composés de type alkylpolyglycosides et/ou des dérivés O-acylés du glucose permettent d'induire et de stimuler la croissance des cheveux et de diminuer leur chute efficacement. Cette demande décrit notamment l'utilisation de dérivés répondant à la formule : 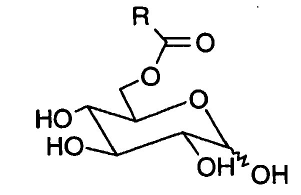 dans laquelle R est une chaîne hydrocarbonée, linéaire, saturée ou insaturée, contenant 7 à 19 atomes de carbone,
pour combattre la chute des cheveux ou stimuler leur croissance, notamment en cosmétique ou en pharmacie.
Ce document mentionne entre autre l'utilisation de dérivés dans lesquels le reste acyle RCO- est un reste octanoyle, décanoyle, dodécanoyle, myristoyle, hexadécanoyle, stéaroyle, oléoyle, linoléoyle ou linolénoyle.
Les radicaux plus particulièrement cités et exemplifiés sont les radicaux octanoyle et oléoyle.

Il reste, d'une manière générale, qu'il serait intéressant et utile de pouvoir disposer de composés actifs autres que ceux déjà connus, de préférence potentiellement plus actifs et/ou moins toxiques, et susceptibles d'être employés dans le domaine cosmétique.

Après de nombreuses recherches, la demanderesse vient de mettre en évidence que, de manière surprenante et inattendue, un nouveau produit bien particulier, dérivé O-acylé du glucose, présentait des propriétés remarquables susceptibles de justifier son utilisation pour améliorer l'état du cheveu, et notamment pour diminuer et/ou freiner la chute des cheveux et/ou des poils, et/ou pour induire et/ou stimuler leur croissance.

En particulier, le produit selon l'invention présente l'avantage d'être composé principalement de dérivés d'acides gras essentiels naturellement présents dans le corps humain.
Ce produit est également plus stable dans le temps que les acides gras libres qu'il comprend.
Enfin, il est aisément synthétisable, à un niveau industriel et avec un coût relativement peu élevé.

Un objet de la présente invention est donc un produit O-acylé dérivé du glucose susceptible d'être obtenu par estérification, partielle ou totale, de la vitamine F avec le glucose.
Un autre objet de l'invention est une composition comprenant, dans un milieu physiologiquement acceptable, au moins un produit tel que ci-dessus défini.
Un autre objet de l'invention est un procédé de traitement cosmétique des cheveux et/ou des poils, dans lequel on applique sur les cheveux et/ou les poils une composition cosmétique telle que ci-dessus définie.
Un autre objet de l'invention est un procédé de traitement cosmétique des cheveux et/ou des poils dans lequel on applique sur les cheveux, les poils, une composition cosmétique comprenant une quantité efficace de produit tel que ci-dessus défini, à laisser celle-ci en contact avec les cheveux et/ou les poils, et éventuellement à rincer.
Un autre objet de l'invention est l'utilisation d'au moins un produit tel que ci-dessus défini, pour la préparation d'une composition, notamment pharmaceutique, destinée à améliorer l'état des poils et/ou des cheveux, et notamment destinée à diminuer et/ou freiner la chute des cheveux et/ou des poils, et/ou pour induire et/ou stimuler leur croissance.

Il est connu de l'homme de l'art que la vitamine F, composé naturellement présent dans les corps gras et notamment dans l'huile de lin, l'huile de tournesol ou l'huile de carthame, est constituée d'un mélange d'acides gras, principalement en C12 à C20.

Ainsi, on considère que la vitamine F est généralement constituée (% en poids) :
- de 75 à 80% en poids d'acide linoléique, et
- de 10 à 15% en poids d'acide oléique,
- de 4 à 8% en poids d'acide palmitique,
- de 0,5 à 3% en poids d'acide stéarique, et
- de 0 à 10% en poids d'un ou plusieurs autres acides choisis parmi les acides laurique, myristique, arachidique, béhénique, lauroleïque, myristoleïque, palmitoleïque et linolénique.

Il en résulte que le produit selon l'invention, qui est susceptible d'être obtenu par estérification de la vitamine F, est donc lui-même constitué d'un mélange de différents esters, découlant de la présence des différents acides, formés lors de cette réaction.
Par ailleurs, le glucose possédant cinq sites susceptibles de réagir lors de la réaction d'estérification, le produit selon l'invention comprend donc également un mélange des esters formés sur les différentes positions du glucose.
Enfin, le produit selon l'invention comprend également un mélange des monoesters et diesters susceptibles d'être formés.
On entendra donc par 'produit selon l'invention', dans la suite de la présente description, le mélange constitué par l'ensemble des esters, mono ou di, formés lors de la réaction d'estérification de la vitamine F et du glucose.

Le produit selon l'invention comprend donc principalement un mélange de composés qui peuvent être représentés par la formule (I) suivante : 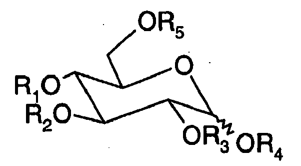 dans laquelle R1, R2, R3, R4 et R5, indépendamment les uns des autres, représentent l'hydrogène ou un radical -CO-R avec R représentant une chaîne hydrocarbonée linéaire, saturée ou insaturée, comprenant 11 à 21 atomes de carbone, sous réserve qu'au moins un des radicaux R1 à R5 soient différents de l'hydrogène.

Le rapport entre le nombre de fonctions esters du produit estérifié et le nombre de fonctions hydroxyles initiales, ou taux d'estérification, pour une molécule de glucose, est de préférence compris entre 0,2 et 1, notamment de 0,2 à 0,6 et en particulier de 0,21 à 0,4.

Par ailleurs, le glucose est estérifié de préférence en position 1, en position 2, en position 3 et/ou en position 6. Préférentiellement, le glucose est estérifié principalement en position 1 et/ou 6.
Le rapport entre le nombre de fonctions esters en position 6 et le nombre total de fonctions esters, pour une molécule de glucose, est de préférence compris entre 0,55 et 1, notamment entre 0,70 et 0,98 et préférentiellement entre 0,90 et 0,97.

Notamment, le produit selon l'invention peut comprendre au moins un ester, notamment monoester, de glucose et d'acide linoléique; au moins un ester, notamment monoester, de glucose et d'acide oléïque; au moins un ester, notamment monoester, de glucose et d'acide palmitique; et au moins un ester, notamment monoester, de glucose et d'acide stéarique.
Il peut en outre comprendre au moins un ester, notamment monoester, de glucose et d'un ou plusieurs esters de glucose et d'acide laurique, myristique, arachidique, béhénique, lauroleïque, myristoleïque, palmitoleïque et/ou linolénique.
Il peut en outre également comprendre au moins un diester de glucose et d'un ou plusieurs acides choisis parmi les acides laurique, myristique, arachidique, béhénique, lauroleïque, myristoleïque, palmitoleïque, linoléique, oléique, palmitique, stéarique et/ou linolénique.

Ainsi, de préférence, le produit selon l'invention comprend généralement, toutes positions confondues, :
- de 40 à 80% en poids, de préférence 60 à 75% en poids, préférentiellement 68-72% en poids, de monoester de glucose et d'acide linoléïque,
- de 10 à 20% en poids, de préférence 12 à 17% en poids, préférentiellement 14-15% en poids, de monoester de glucose et d'acide oléique,
- de 5 à 20% en poids, de préférence 7 à 15% en poids, préférentiellement 9-12% en poids, de monoester de glucose et d'acide palmitique,
- de 0,5 à 7% en poids, de préférence 1 à 5% en poids, préférentiellement 2-4% en poids, de monoester de glucose et d'acide stéarique,
- de 0 à 10% en poids, notamment 0,10-4% en poids, voire 0,15-2% en poids, d'un ou plusieurs monoesters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

En particulier, le produit selon l'invention peut comprendre :
- de 40 à 80% en poids, de préférence 60 à 75% en poids, préférentiellement 68-72% en poids, d'ester de glucose et d'acide linoléique dont principalement le 6-O-octadeca-9,12-dienoyl-D-glucopyranose, le 1-O-octadeca-9,12-dienoyl-D-glucopyranose, le 2-O-octadeca-9,12-dienoyl-D-glucopyranose et/ou le 3-O-octadeca-9,12-dienoyl-D-glucopyranose,
- de 10 à 20% en poids, de préférence 12 à 17% en poids, préférentiellement 14-15% en poids, d'ester de glucose et d'acide oléique, dont principalement le 6-O-octadeca-9-enoyl-D-glucopyranose, le 3-O-octadeca-9-enoyl-D-glucopyranose, le 1-O-octadeca-9-enoyl-D-glucopyranose et/ou le 2-O-octadeca-9-enoyl-D-glucopyranose,
- de 5 à 20% en poids, de préférence 7 à 15% en poids, préférentiellement 9-12% en poids, d'ester de glucose et d'acide palmitique, dont principalement le 6-O-hexadecanoyl-D-glucopyranose, le 3-O-hexadecanoyl-D-glucopyranose, le 1-O-hexadecanoyl-D-glucopyranose et/ou le 2-O-hexadecanoyl-D-glucopyranose.
- de 0,5 à 7% en poids, de préférence 1 à 5% en poids, préférentiellement 2-4% en poids, d'ester de glucose et d'acide stéarique, dont principalement le 6-O-octadecanoyl-D-glucopyranose, le 3-O-octadecanoyl-D-glucopyranose, le 1-O-octadecanoyl-D-glucopyranose et/ou le 2-O-octadecanoyl-D-glucopyranose,
- de 0 à 10% en poids, notamment 0,10-4% en poids, voire 0,15-2% en poids, d'un ou plusieurs esters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléique, palmitique, stéarique et/ou linolénique.

La réaction d'estérification peut notamment être effectuée selon toutes méthodes connues. La synthèse peut notamment être réalisée à partir du chlorure d'acide (chlorure de vitamine F) et du D-glucose, selon la méthode décrite par Reinfeld et al. dans "Die Stärke" n°6, pages 181-189, 1968.
En effet, plusieurs méthodes d'estérification du D-glucose avec l'acide laurique ont été décrites et comparées dans la revue "Die Stärke", n°6, p.181-189 en 1968 par E. REINEFELD.
Parmi les agents d'acylation du glucose, il a été proposé d'employer des chlorures d'acides, des immidazolides d'acides, des anhydrides mixtes carboxyliques-carboniques et des anhydrides carboxyliques. Dans le cas des réactions de transestérification, les agents employés sont les esters méthyliques ou éthyliques des acides.
Il ressort de cette publication que la méthode permettant l'obtention du rendement le plus élevé est l'acylation à l'aide du chlorure d'acide. Avec le chlorure de lauroyle, on obtient par exemple un mélange de monoester et de diesters, avec un rendement de 49% dont 36% pour le monoester.
Cette méthode a notamment été utilisée dans le brevet EP0485251, pour conduire aux 6-O-acyles glucose avec un rendement, par exemple, de 40% avec le chlorure d'oléyle.

Toutefois, il n'est pas toujours aisé de disposer du chlorure d'acide adéquat. En l'absence de chlorure d'acide industriel, il est alors nécessaire d'utiliser une autre méthode.
L'acylation employant des imidazolides d'acide conduit à un mélange de monoester et de diesters, avec un rendement total de 22% et un rendement de 9% pour le monoester seul, lorsque l'on utilise l'imidazolide d'acide laurique.
L'acylation du glucose par le biais de la formation d'anhydride vrai conduit aux composés recherchés avec un rendement total de 46% pour le mélange monoester et diesters, et de 28% pour l'obtention du monoester.
Toutefois ce procédé génère la formation d'acides gras libres qu'il est nécessaire d'éliminer pour conduire à des produits terminaux relativement purs.
Or, cette élimination peut s'avérer parfois difficile, au vu la nature des impuretés. Par ailleurs, on cherche généralement à éviter les étapes intermédiaires de purification, qui rallongent inutilement le procédé et qui engendrent des coûts supplémentaires, ceci étant incompatible avec un procédé au niveau industriel.
On a constaté que, quelle que soit la méthode envisagée, l'agent d'acylation choisi et/ou la proportion de chacun des réactifs, l'acylation du glucose conduisait toujours à l'obtention d'un mélange dans lequel on a pu identifier le D-glucopyranose-6-ester, mais également le D-glucopyranose-1,6-diester et le D-glucopyranose-2,6-diester comme produits de réaction coexistants.
Il subsiste donc le besoin de disposer d'une nouvelle voie de synthèse des dérivés O-acylés du glucose, permettant l'obtention de ces composés d'une manière rapide et aisée, au niveau industriel, avec un rendement en produits recherchés important.

La présente invention a également pour but de pallier les inconvénients de l'art antérieur et de proposer un tel procédé qui permet la préparation desdits dérivés O-acylés du glucose avec un rendement de l'ordre de 70% minimum.
Par ailleurs, on a constaté que ce nouveau procédé permet également l'obtention de manière sélective des esters de glucose majoritairement en position 6.
En outre, ce procédé permet l'emploi de chlorures d'acide industriels.

La présente invention a donc pour objet un procédé de préparation de dérivés O-acylés majoritairement en position 6 du glucose de formule (la) : 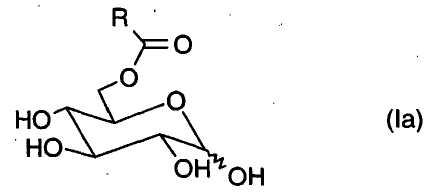 dans laquelle R est une chaîne hydrocarbonée linéaire ou ramifiée, saturée ou insaturée, comprenant 7 à 21 atomes de carbone, comprenant des étapes consistant:
- dans une première étape, à préparer un anhydride mixte de formule (ll): 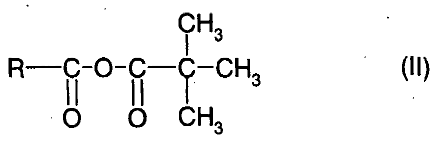 par réaction d'un acide carboxylique de formule R-COOH avec un halogénure d'acide triméthyle acétique de formule X-C(O)-C(CH₃)₃, avec X représentant de préférence le chlore ou le brome, et
- dans une seconde étape, à faire réagir ledit anhydride mixte formé avec le glucose.

Le procédé objet de la présente invention permet de préparer notamment des dérivés O-acylés du glucose majoritairement en position 6, seuls ou en mélange, qui peuvent être représentés par la formule (la). Le procédé selon l'invention permet en particulier de préparer le produit O-acylé dérivé du glucose susceptible d'être obtenu par estérification, partielle ou totale, du glucose et de la vitamine F.

De préférence, le radical R est une chaîne hydrocarbonée linéaire ou ramifiée, saturée ou insaturée, comprenant 11 à 17 atomes de carbone.
Le reste acyle -COR peut notamment être un reste octanoyle, décanoyle, dodécanoyle, myristoyle, hexadécanoyle, stéaroyle, palmitoléoyle, oléoyle, linoléoyle ou linolénoyle.

Le procédé selon l'invention consiste donc, dans une première étape, à préparer un anhydride mixte de formule (II), par réaction d'un acide carboxylique de formule R-COOH avec un halogénure d'acide triméthyle acétique de formule X-C(O)-C(CH₃)₃, avec X représentant de préférence le chlore ou le brome.
Parmi les acides carboxyliques susceptibles d'être employés, on peut citer les acides octanoïque, décanoïque, dodécanoïque, myristique, hexadécanoïque, stéarique, oléique, linoléique ou linolénique, et leurs mélanges.
Le schéma réactionnel est alors le suivant : 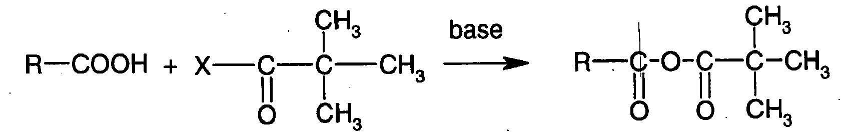 La réaction peut être effectuée dans un solvant organique réactionnel, tel que le tétrahydrofuranne, le N,N-diméthylformamide, le N-méthylpyrrolidone, la pyridine, le toluène et leurs mélanges.
De préférence, elle peut être effectuée sous atmosphère inerte, azote par exemple.
On peut employer une base pour activer l'acide carboxylique, ou bien utiliser directement le carboxylate correspondant; cette base est, de préférence, une base organique notamment choisie parmi la triéthylamine, la pyridine, la 4-diméthylaminopyridine, la tributylamine, la N-méthylmorpholine et leurs mélanges.
La réaction peut être effectuée à une température de -25°C à +40°C, de préférence, -10°C à +10°C, et pendant une durée de 5 minutes à 5 heures, notamment de 30 minutes à 3 heures.
De préférence, on fait réagir 0,3 à 3 équivalents, de préférence 0,5 à 1,5 équivalents, d'acide carboxylique avec 1 équivalent d'halogénure d'acide triméthyle acétique.

Dans la seconde étape du procédé, étape d'estérification, on fait réagir ledit anhydride mixte avec du glucose. Cette seconde étape peut être éventuellement réalisée après essorage des sels éventuellement formés lors de la première étape.
Cette seconde étape est de préférence effectuée dans un solvant organique, qui peut être le même solvant organique que celui de la première étape. Ce solvant peut donc être le tétrahydrofuranne, le N,N-diméthylformamide, le N-méthylpyrrolidone, la pyridine, le toluène et leurs mélanges.
De préférence, l'anhydride mixte est mis en solution dans ledit solvant organique, avant réaction.

De préférence, le glucose est préalablement mis en solution dans un solvant tel que la pyridine, le diméthylformamide, la N-méthylpyrrolidone et/ou le diméthylacétamide.
De préférence, on fait réagir 0,5 à 1,5, notamment 0,9 à 1,1, et encore mieux 1, équivalents d'anhydride mixte avec 3 équivalents de glucose.
On utilise de préférence, selon l'invention, au moins 3 équivalents de glucose par rapport à l'acide ou au mélange d'acides, mis à réagir dans la première étape.
La réaction peut être effectuée à une température de -25°C à +100°C, de préférence, 0°C à +60°C, encore mieux à 20-25°C, et pendant une durée de 1 à 30 heures, notamment de 2 à 15 heures.
Après la fin de la réaction, les solvants peuvent être séparés du composé recherché, par exemple par évaporation, centrifugation ou filtration.
Le produit résultant peut être nettoyé par tout moyen connu, tel que distillation, chromatographie sur colonne de gel de silice, précipitation et/ou extraction par exemple par un mélange eau/solvants organiques.

Le procédé selon l'invention permet donc de préparer de manière industriellement réalisable, des dérivés O-acylés du glucose, majoritairement en position 6, seuls ou en mélange. En particulier, on peut préparer selon ce procédé les composés suivants : le 6-O-octadeca-9,12-dienoyl-D-glucopyranose; le 6-O-octadeca-9-enoyl-D-glucopyranose; le 6-O-octadecanoyl-D-glucopyranose; le 6-O-hexadecanoyl-D-glucopyranose; et leurs mélanges.
On a constaté que, d'une manière générale, avec le procédé selon la présente invention, le glucose était estérifié principalement en position 6, et éventuellement en position 1 et/ou 2 et/ou 3 également.
Le rapport entre le nombre de fonctions esters en position 6 et le nombre total de fonctions esters, pour une molécule de glucose, est généralement compris entre 55 et 95%, notamment entre 60 et 80%, préférentiellement de 68-75%.

Le produit O-acylé dérivé du glucose selon l'invention peut être notamment employé dans une composition ou pour la préparation d'une composition qui comprend par ailleurs un milieu physiologiquement acceptable. Cette composition peut notamment se présenter sous la forme d'une composition cosmétique qui comprend donc un milieu cosmétiquement acceptable ou sous la forme d'une composition pharmaceutique qui comprend donc un milieu pharmaceutiquement acceptable.
La quantité de produit à utiliser dans les compositions selon l'invention peut être aisément déterminée par l'homme du métier, notamment selon la nature de la composition et/ou l'effet recherché.
D'une manière générale, cette quantité peut être comprise entre 0,01-20 % en poids par rapport au poids total de la composition, notamment entre 0,1-10% en poids, et de préférence entre 0,5-5% en poids.

Le milieu physiologiquement, cosmétiquement ou pharmaceutiquement, acceptable dans lequel le produit selon l'invention peut être employé est aisément déterminable par l'homme du métier. Il s'agit d'un milieu compatible avec une application sur les matières kératiniques telles que les cils, les sourcils, les poils et/ou les cheveux.
D'une manière générale, il peut être anhydre ou aqueux.
On entend par milieu anhydre, un milieu solvant contenant moins de 1% en poids d'eau. Ce milieu peut être constitué d'un solvant organique ou d'un mélange de solvants organiques choisis plus particulièrement parmi les alcools en C₁-C₄ comme l'éthanol; les alkylèneglycols comme le propylèneglycol; les alkyléthers d'alkylèneglycols ou de dialkylèneglycols, dont les radicaux alkyle ou alkylène contiennent de 1 à 4 atomes de carbone.
On entend par milieu aqueux, un milieu constitué par de l'eau ou par un mélange d'eau et d'un autre solvant physiologiquement acceptable, choisi notamment parmi les solvants organiques cités ci-dessus. Dans ce dernier cas, ces autres solvants peuvent représenter environ 5 à 95% en poids de la composition.

Il est possible d'ajouter dans la composition, en association avec le dérivé selon l'invention, un ou plusieurs actifs cosmétiques ou pharmaceutiques, tels que, notamment :
- des composés améliorant l'activité sur la repousse et/ou sur le freinage de la chute des cheveux, et ayant déjà été décrits pour cette activité, comme par exemple les esters d'acide nicotinique, dont notamment le nicotinate de tocophérol, le nicotinate de benzyle et les nicotinates d'alkyles en C₁-C₆ comme les nicotinates de méthyle ou d'hexyle; les dérivés de pyrimidine, comme le 2,4-diamino 6-piperidinopyrimidine 3- oxyde ou "Minoxidil" décrit dans les brevets US 4 139 619 et US 4 596 812 ; les dérivés de pyrimidine 3-oxyde comme ceux décrits dans WO92/01437 ou WO96/09048, et notamment l"'Aminexil" ou 2,4-diaminopyrimidine 3-N-oxyde;
- les agents antibactériens tels que les macrolides, les pyranosides et les tétracyclines, et notamment l'érythromycine ;
- les agents antagonistes de calcium, comme la cinnarizine, le diltiazem, la nimodipine et la nifedipine ;
- des hormones telles que l'estriol et ses analogues, la vitamine D et ses analogues, ou la thyroxine et ses sels ;
- des agents anti-inflammatoires stéroïdiens ou non stéroidiens, tels que les corticostéroïdes (par exemple : l'hydrocortisone) ;
- des agents antiandrogènes, tels que l'oxendolone, la spironolactone, le diéthylstilbestrol et la flutamidem ;
- des inhibiteurs stéroïdiens ou non stéroïdiens des 5-α-réductases tels que le finastéride ou l'acide 4,6-diméthoxy-indole 2-carboxylique ou ses dérivés tels que décrits dans EP1068858;
- des agonistes potassiques tels que la cromakalim et le nicorandil.
- des agonistes des récepteurs de RXR des rétinoïdes et des antagonistes des rétinoïdes ;
- des capteurs de radicaux OH, tels que le diméthylsulfoxyde ;
- des peptides comme par exemple le tripeptide Lys-Pro-Val, et plus généralement l'α-MSH et ses dérivés;
- des antipelliculaires ou antifongiques tels que le Zinc Pyrithione, la Piroctone Olamine, le disulfure de sélénium, la Tropolone, l'Hinokitiol, les complexes hinokitiol-zinc et hinokitiol-cuivre notamment décrits dans EP0728478, les complexes métalliques divalents tels que ceux décrits dans FR01-03309.
- des agents anti-radicaux libres; des antiséborrhéiques; des antiparasitaires; des antiviraux; des agents antiprurigineux.

On peut ajouter à cette liste, les composés suivants : le diazoxyde, la spiroxasone, des phospholipides comme la lécithine, les acides linoléique et linolénique; l'acide salicylique et ses dérivés décrits dans le brevet français FR2581542, comme les dérivés de l'acide salicylique porteurs d'un groupement alcanoyle ayant de 2 à 12 atomes de carbone en position 5 du cycle benzénique; des acides hydroxycarboxyliques ou cétocarboxyliques et leurs esters; des lactones et leurs sels correspondants; l'anthraline, des caroténoides; les acides eicosatétrayénoïque et eicosatriyénoïque ou leurs esters et amides; des extraits d'origine végétale ou bactérienne.

D'autre part, on peut ajouter au milieu physiologiquement acceptable, des adjuvants habituellement utilisés dans le domaine d'application considéré, notamment dans le domaine cosmétique, tels que des tensioactifs, des émulsionnants, des épaississants ou gélifiants hydrophiles ou lipophiles, des agents cosmétiques, des conservateurs, des solvants, des antioxydants, des filtres UV, des agents alcalinisants ou acidifiants; des huiles et/ou des cires d'origine animale, minérale, végétale ou de synthèse; des charges, des matières colorantes telles que des pigments et/ou des colorants; bien connus dans l'état de la technique.
La nature et la quantité de ces adjuvants peuvent être choisies par l'homme du métier, sur la base de ses connaissances générales, de manière à obtenir la forme de présentation désirée pour la composition. En tout état de cause, l'homme du métier veillera à choisir tous les éventuels composés complémentaires et/ou leur quantité, de manière telle que les propriétés avantageuses de la composition selon l'invention ne soient pas, ou substantiellement pas, altérées par l'adjonction envisagée.

Comme huiles ou cires utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles ou cires siliconées (cyclométhicone) et les huiles fluorées (perfluoropolyéthers), les cires d'abeille, de carnauba ou paraffine. On peut ajouter à ces huiles des alcools gras et des acides gras (acide stéarique).
Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose 63 par la société Gattefosse.

Comme solvants utilisables dans l'invention, on peut citer les alcools inférieurs, notamment l'éthanol et l'isopropanol, le propylène glycol.

Comme gélifiants hydrophiles utilisables dans l'invention, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, éthylcellulose, polyéthylène.

Les compositions utilisables selon l'invention peuvent contenir d'autres actifs cosmétiques ou pharmaceutiques hydrophiles comme les protéines ou les hydrolysats de protéine, les acides aminés, les polyols, l'urée, l'allantoïne, les sucres et les dérivés de sucre, les vitamines hydrosolubles, les extraits végétaux et les hydroxyacides.
Comme actifs cosmétiques ou pharmaceutiques lipophiles, on peut utiliser le rétinol (vitamine A) et ses dérivés, le tocophérol (vitamine E) et ses dérivés, les acides gras essentiels, les céramides, les huiles essentielles, l'acide salicylique et ses dérivés.

Les compositions selon l'invention peuvent se présenter sous toutes les formes galéniques normalement utilisées.
La composition peut avoir la forme notamment de solution aqueuse ou huileuse ou de dispersion du type lotion ou sérum; d'émulsion de consistance liquide ou semi-liquide du type lait, de type huile-dans-eau, eau-dans-huile ou multiple; de suspension ou émulsion de consistance molle du type crème ou gel aqueux ou anhydre; de microcapsule ou microparticule; de dispersion vésiculaire de type ionique et/ou non ionique. Elle peut être également utilisée sous forme de solution aqueuse, alcoolique ou hydroalcoolique, ou sous forme de crème, de gel, d'émulsion, de mousse ou encore sous forme de composition pour aérosol comprenant également un agent propulseur sous pression.

La composition selon l'invention peut être une composition pour soins capillaires, et notamment un shampooing, une lotion ou une crème traitante, une lotion de mise en plis, une crème ou un gel coiffant; une composition de teinture, notamment teinture d'oxydation, éventuellement sous forme de shampooing colorant; une lotion restructurante pour les cheveux, une composition de permanente (notamment une composition pour le premier temps d'une permanente), une lotion ou un gel antichute, un shampooing antiparasitaire.

Les compositions selon l'invention peuvent être appliquées sur les zones alopéciques d'un individu, puis être éventuellement laissées en contact plusieurs heures et être éventuellement rincées.
On peut, par exemple, appliquer la composition comprenant une quantité efficace de produit selon l'invention, le soir; garder celle-ci au contact toute la nuit et éventuellement effectuer un shampooing le matin.

La présente invention à également pour objet un procédé de traitement cosmétique des cheveux et/ou des poils, dans lequel on applique sur la peau, les cheveux et/ou les poils, une composition cosmétique comprenant une quantité efficace de produit selon l'invention, à laisser celle-ci en contact avec les cheveux et/ou les poils, et éventuellement à rincer.
Le procédé de traitement présente les caractéristiques d'un procédé cosmétique dans la mesure où il permet d'améliorer l'esthétique des poils et/ou des cheveux, notamment en leur donnant une plus grande vigueur et/ou un aspect amélioré.

L'invention est illustrée plus en détail dans les exemples suivants.
Dans ces exemples, les composés 6-O-octadeca-9-enoyl-D-glucopyranose et 6-O-hexadecanoyl-D-glucopyranose ont été préparés selon la méthode décrite dans le brevet EP485251.
Les composés 6-O-octadeca-9,12-dienoyl -D-glucopyranose et 6-O-octadecanoyl-D-glucopyranose sont décrits dans la littérature.

### Exemple 1 : préparation de l'ester de glucose de la vitamine F (majoritairement ester en position 6)

Dans un tricol de 500 ml, on dilue 17 ml de chlorure d'acide triméthyle acétique dans 100 ml de tétrahydrofurane; on ajoute, sous atmosphère inerte et à 0°C, un mélange de 37,3 g de vitamine F et 19,3 ml de triéthylamine préalablement mis en solution dans 100 ml de tétrahydrofurane; on laisse sous agitation pendant 1 heure puis on filtre les sels formés pour obtenir une solution.
Dans un tricol de 2 litres, on dissout 96 g de D-glucose dans 1,15 litre de pyridine, et on y ajoute la solution précédente, sous atmosphère inerte, à température ambiante. On laisse le mélange sous agitation pendant une nuit.
Le milieu réactionnel est évaporé à sec, sous vide pour éliminer la pyridine, puis la pâte obtenue est extraite (mélange eau/solvant organique) et la phase organique récupérée est séchée, filtrée et évaporée.
On obtient 49 g d'une pâte jaune (rendement : 83 %) d'ester de vitamine F, dont 72% de monoesters (mélange) en position 6.
Spectre RMN ¹H (DMSO) 200MHz : δ (ppm) : 0,85; 1,23; 1,50; 2,00; 2,26; 2,73; 3,03; 3,13; 3,40; 3,76; 3,97; 4,25; 4,53; 4,76; 4,89; 5,04; 5,32; 6,34.
Spectre RMN ¹³C (DMSO) 200MHz : δ (ppm) : 13,95; 22,12; 24,48; 25,23; 26,62; 28,46 à 29,08; 31,32; 33,44; 63,91; 69,14; 70,57; 72,19; 72,86; 92,30; 127,77; 129,73; 172,92.
Les spectres RMN ¹H et ¹³C (DMSO) 200MHz sont conformes à la structure attendue.

### Exemple 2 : Préparation de l'ester de glucose de la vitamine F (majoritairement ester en position 3)

Dans un ballon de 500 ml, sous atmosphère d'azote, on introduit 20 g de vitamine F solubilisée dans 300 ml de toluène anhydre auquel on additionne 3 gouttes de DMF pour catalyser la réaction. On ajoute ensuite goutte à goutte 12,6 ml de chlorure d'oxalyle (dégagement gazeux) et on laisse sous agitation à 25°C pendant 3 heures. Le milieu réactionnel est concentré au maximum, puis dilué dans 200 ml de dichlorométhane. On obtient ainsi le chlorure de vitamine F utilisé dans l'étape suivante.
Dans un tricol de 500 ml surmonté d'un réfrigérant et d'une ampoule à brome, sous atmosphère d'azote, on introduit 29,6 g de diacétone-D-glucose solubilisée dans 200 ml de dichlorométhane, et 26 ml de triéthylamine.
On maintient la température à 10°C environ à l'aide d'un bain d'eau glacée.
On ajoute goutte à goutte 200 ml du chlorure de vitamine F ci-dessus tout en maintenant la température à environ 10°C. Puis le milieu réactionnel est laissé 2 heures à température ambiante, sous agitation.
Le mélange pâteux obtenu est dilué par ajout de 200 ml de dichlorométhane. On réalise ensuite plusieurs lavages : (i) ajout d'eau distillée et élimination de la solution aqueuse supérieure, (ii) ajout d'une solution d'acide chlorhydrique 1 N et élimination de la phase aqueuse, (iii) ajout d'eau distillée et élimination de la phase aqueuse.
La phase organique est séchée sur sulfate de sodium puis filtrée et concentrée à sec.
On obtient une huile épaisse marron clair qui est solubilisée dans 350 ml d'un mélange eau/acide trifluoroacétique (à 11.10⁻³ mol/litre) et laissée à température ambiante pendant 1 heure. Le mélange est concentré puis repris 5 fois avec 100 ml de toluène. Le résidu est purifié sur gel de silice.
On obtient 12 g de composé sous la forme d'une poudre jaune. RMN ¹³C (DMSO) 200MHz : δ (ppm) : 60,76; 63,82; 92,10; 92,24; 96,75; 96,86
Les spectres RMN ¹H et ¹³C (DMSO) 200MHz sont conformes à la structure attendue.

### Exemple 3 : Préparation du 3-O-octadeca-9,12-dienoyl -D-glucopyranose

Dans un tricol de 500 ml surmonté d'un réfrigérant et d'une ampoule à brome, sous atmosphère d'azote, on introduit 29,6 g de diacétone-D-glucose solubilisée dans 200 ml de dichlorométhane, et 26 ml de triéthylamine.
On maintient la température à 10°C environ à l'aide d'un bain d'eau glacée.
On ajoute goutte à goutte 200 ml de chlorure d'acide octadéca-9,12-diénoïque (linoléïque) tout en maintenant la température à environ 10°C. Puis le milieu réactionnel est laissé 2 heures à température ambiante, sous agitation.
Le mélange pâteux obtenu est dilué par ajout de 200 ml de dichlorométhane. On réalise ensuite plusieurs lavages : (i) ajout d'eau distillée et élimination de la solution aqueuse supérieure, (ii) ajout d'une solution d'acide chlorhydrique 1N et élimination de la phase aqueuse, (iii) ajout d'eau distillée et élimination de la phase aqueuse.
La phase organique est séchée sur sulfate de sodium puis filtrée et concentrée à sec.
On obtient 21 g d'une huile épaisse marron clair qui est solubilisée dans 350 ml d'un mélange eau/acide trifluoroacétique (à 11.10⁻³ mol/litre) et laissée à température ambiante pendant 1 heure. Le mélange est concentré puis repris 5 fois avec 100 ml de toluène. Le résidu est purifié sur gel de silice.
On obtient 10,8 g de composé sous la forme d'une huile jaune (rendement 64%).

Les spectres RMN ¹H et ¹³C (DMSO) 200MHz sont conformes à la structure attendue.

### Exemple 4

On sait qu'une solution pour freiner la propagation du processus qui conduit à la chute excessive des cheveux est d'employer des actifs cosmétiques visant à limiter la synthèse de médiateurs issus de la voie de la 5-lipoxygénase, à l'exemple des leucotriènes. Les inhibiteurs de lipoxygénases entrent dans la catégorie de ces actifs.
On a donc testé l'effet des composés selon l'invention sur l'inhibition de la 15-lipoxygénase de soja purifiée, sachant que la 15-lipoxygénase de soja est un modèle d'étude couramment utilisé comme modèle prédictif de la 5-lipoxygénase humaine.

Le test mis en oeuvre est le suivant:
- préparation d'une solution-tampon Tris 100 mM, pH= 7,5
- préparation d'une suspension de 15-lipoxygénase L1 purifiée de soja (0,20 mg/ml) dans un tampon Tris 100 mM, pH= 7,5; stockage dans la glace;
- calibration de l'oxymètre, cuve munie d'un système d'agitation magnétique et thermostatée à 25°C;
- préparation d'une solution hydroalcoolique (éthanol absolu/eau, 5/95, v/v) d'arachidonate de potassium à 7,5 mM;
- préparation d'une solution du composé à tester à 30 mM dans le diméthyle sulfoxyde (DMSO);
- introduction dans la cuve de l'oxymètre, sous agitation, de 456,6 µl de solution-tampon, de 16,7 µl de solution d'arachidonate de potassium, et de 16,7 µl de solution du composé à tester ou de 16,7 µl de DMSO dans le cas de la mesure "témoin".
L'enregistreur est déclenché et 10 µl de suspension de 15-lipoxygénase sont introduits dans la cuve.
La vitesse maximale de consommation d'oxygène (Vmax) est enregistrée.
Les différentes Vmaxs sont comparées à celle du témoin et les résultats sont exprimés en % de celle-ci. Le test est répété 10 fois et l'on fait une moyenne sur ces 10 essais.

On teste les composés suivants :
A : 6-O-octadeca-9,12-dienoyl -D-glucopyranose
B : 6-O-octadeca-9-enoyl-D-glucopyranose
C : 6-O-octadecanoyl-D-glucopyranose
D : 6-O-hexadecanoyl-D-glucopyranose
E : 3-O-octadeca-9,12-dienoyl -D-glucopyranose
F : ester de glucose de la vitamine F (majoritairement ester en position 6)
G : ester de glucose de la vitamine F (majoritairement ester en position 3)

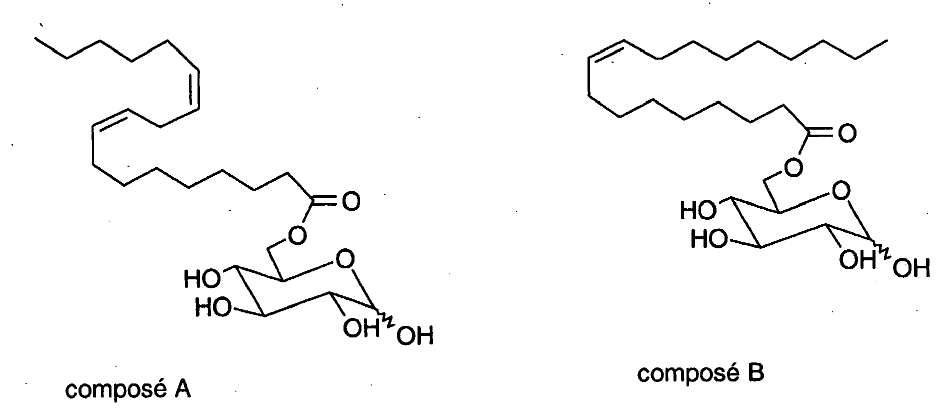

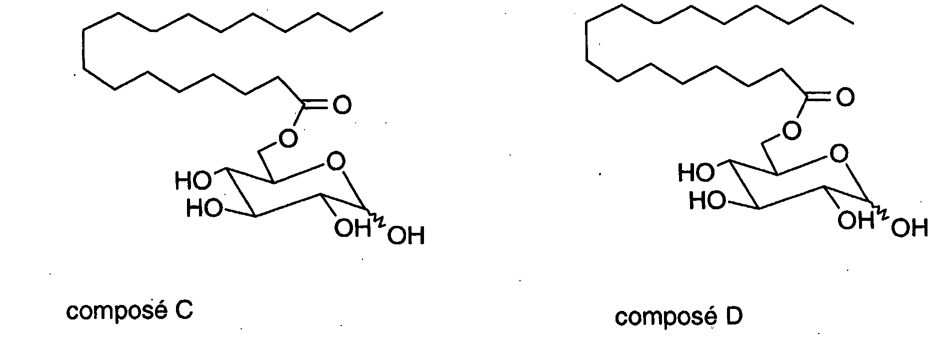

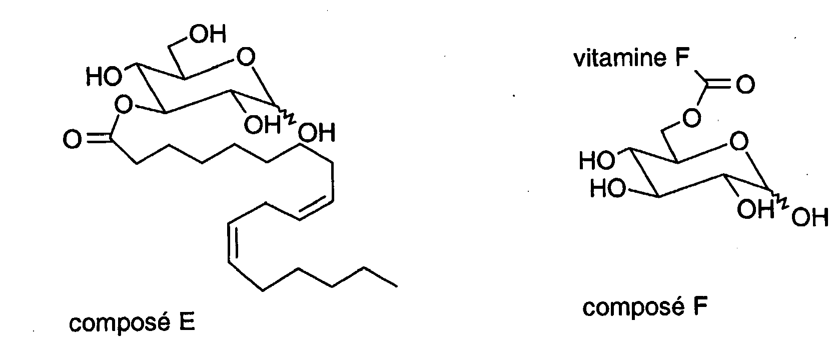

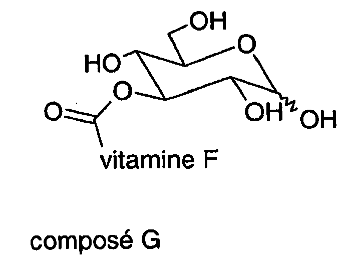

On obtient les résultats suivants, exprimé en % d'inhibition du composé testé (à 1 mM) par rapport au témoin :

| Composé | % d'inhibition |
|---|---|
| Composé A | 71% |
| Composé B | 30% |
| Composé C | 49% |
| Composé D | 15% |
| Composé E | 80% |
| Composé F | 81% |
| Composé G | 86% |

On constate donc que les produits selon la présente invention (composés F et G) présentent une bonne capacité d'inhibition de lipoxygénase de soja.
Cette capacité est même supérieure à celle des esters de glucose et d'acide en C18, saturé ou non, ainsi qu'à celle de l'ester en C16 saturé.
Or, ceci est particulièrement surprenant étant donné qu'on aurait pu s'attendre à ce que le mélange "ester en C16 + esters en C18" soit moins actif que les esters en C18 seuls.

### Exemple 5

On a évalué la stabilité des composés selon l'invention (mesure de l'hydrolyse des esters).
On prépare des solutions à 0,1% en poids des composés dans un mélange éthanol/isopropanol/eau (64/16/20 en volume). On laisse ces solutions dans une enceinte thermostatée à 45°C, pendant 2 mois.
On détermine ensuite par HPLC, le pourcentage de linoléate de glucopyranose hydrolysé.

On obtient les résultats suivants :

| Composé | % d'hydrolyse |
|---|---|
| ester de glucose de la vitamine F (majoritairement ester en position 6) | 3% |
| 6-O-octadeca-9,12-dienoyl -D-glucopyranose | 7% |
| ester de glucose de la vitamine F (majoritairement ester en position 3) | 17% |
| 3-O-octadeca-9,12-dienoyl -D-glucopyranose | 30% |

On constate donc que les esters de glucose de la vitamine F présentent une stabilité meilleure que celle des esters de glucose de l'acide linoléïque.
Par ailleurs, la stabilité de l'ester en position 6 de glucose et de la vitamine F, est légèrement meilleure que celle du même ester estérifié en position 3.

### Exemple 6: Lotion quotidienne

On prépare une composition comprenant les constituants suivants :
- composé de l'exemple 1 0,05 g
- éthanol 60 g
- parfum, colorants qs
- eau déminéralisée qsp 100 g

### Exemple 7 : Gel Liposomé

On prépare une composition comprenant les constituants suivants :
- composé de l'exemple 2 0,5 g
- Carbomer 0,25 g
- triéthanolamine qs pH 7
- conservateurs qs
- eau déminéralisée qsp 100 g

### Exemple 8 : Lotion antichute

On prépare une composition comprenant les constituants suivants :
- composé de l'exemple 1 1 g
- propylène glycol 10 g
- isopropanol qsp 100 g

On applique 1 ml de cette lotion à la fréquence de une à deux fois par jour.

### Exemple 9 : Lotion antichute

On prépare une composition comprenant les constituants suivants :
- composé de l'exemple 2 2 g
- propylène glycol 30 g
- éthanol 40,5 g
- eau qsp 100 g

On applique cette lotion une à deux fois par jour, à raison de 1 ml par application.

### Exemple 10 : Lotion antichute

On prépare une composition comprenant les constituants suivants :
- composé de l'exemple 2 1 g
- monométhyléther de propylèneglycol (Dowanol PM de Dow) 20 g
- hydroxypropylcellulose (Klucel G de Herculès) 3 g
- éthanol 40 g
- eau qsp 100 g

On applique cette lotion épaissie une à deux fois par jour, à raison d'un ml par application.

### Exemple 11 : Lotion antichute

On prépare une composition comprenant les constituants suivants :
- composé de l'exemple 1 0,2 g
- dérivé de pyrimidine 3-oxyde (Aminexil) 1,5 g
- eau qsp 100 g

### Exemple 12 : Shampooing antipelliculaire

On prépare un shampooing comprenant :
- Composé de l'exemple 1 1,2 g
- Acide Salicylique 2 g
- Polyglyceryl 3-hydroxylaurylether 26 g M.A.
- Hydroxy propyl cellulose (Klucell G de HERCULES) 2 g
- Conservateur 50 g
- Triéthanolamine qs pH 7,5
- Eau qsp 100 g

Ce shampooing est utilisé quotidiennement à raison de 10 g par chevelure avec un temps de pose de l'ordre d'une minute et ceci pendant une période de 2 semaines. On observe alors une chute rapide des sensations de prurit et une nette amélioration de l'état pelliculaire.

### Exemple 13 : Lotion anti-pelliculaire

On prépare une composition comprenant :
- Composé de l'exemple 2 0,3 g M.A.
- Octopirox (Piroctone Olamine) 0,2 g
- Ethanol 30 g M.A.
- Eau qsp 100 g

Cette solution est appliquée quotidiennement à raison de 6 ml et ce pendant 1 à 2 semaines. On constate alors une amélioration notable de l'état pelliculaire.

### Exemple 14 : préparation du 6-O-oleoyl-glucose

Dans un tricol de 250 ml, on introduit 6,02 g de chlorure d'acide triméthyle acétique et 50 ml de tétrahydrofurane. On ajoute lentement, sous atmosphère inerte et à 0°C, un mélange de 14,1 g d'acide oléique et 5,05 g de triéthylamine dilués dans 50 ml de tétrahydrofurane. On laisse sous agitation pendant 1 heure puis on filtre les sels formés pour obtenir une solution.
Dans un tricol de 1 litre, on dissout 36 g de glucose dans 400 ml de pyridine, et on y ajoute la solution précédente, sous atmosphère inerte, à température ambiante; on maintient sous agitation pendant une nuit.

Le milieu réactionnel est évaporé à sec, sous vide pour éliminer la pyridine, puis la pâte obtenue est extraite (mélange eau/solvant organique) et la phase organique récupérée est séchée, filtrée et évaporée. On obtient 19,5 g d'un solide blanc (rendement 87%) de O-oleoyl-glucose.

Les analyses RMN ¹³C et spectre de masse sont conformes à la structure attendue.

### Exemple 15 : préparation du 6-O-linoleovl-glucose

Dans un tricol de 250 ml, on introduit 6,02 g de chlorure d'acide triméthyle acétique et 50 ml de tétrahydrofurane. On ajoute lentement, sous atmosphère inerte et à 0°C, un mélange de 14 g d'acide linoléique et 5,05 g de triéthylamine dilués dans 50 ml de tétrahydrofurane. On laisse sous agitation pendant 1 heure puis on filtre les sels formés pour obtenir une solution.
Dans un tricol de 1 litre, on dissout 36 g de glucose dans 400 ml de pyridine, et on y ajoute la solution précédente, sous atmosphère inerte, à température ambiante; on maintient sous agitation pendant une nuit.
Le milieu réactionnel est évaporé à sec, sous vide pour éliminer la pyridine, puis la pâte obtenue est extraite (mélange eau/solvant organique) et la phase organique récupérée est séchée, filtrée et évaporée.
On obtient 18,7 g d'une pâte jaune (rendement 82%) de O-linoleoyle-glucose. Le rendement en 6-O-linoleoyle-glucose est, quant à lui, de 62%.

Les analyses RMN ¹³C et spectre de masse sont conformes à la structure attendue.

## Revendications

1. Produit O-acylé dérivé du glucose susceptible d'être obtenu par estérification, partielle ou totale, de la vitamine F avec le glucose, ladite vitamine F étant constituée, en % en poids :
- de 75 à 80% en poids d'acide linoléïque,
- de 10 à 15% en poids d'acide oléïque,
- de 4 à 8% en poids d'acide palmitique,
- de 0,5 à 3% en poids d'acide stéarique, et
- de 0 à 10% en poids d'un ou plusieurs autres acides choisis parmi les acides laurique, myristique, arachidique, béhénique, lauroleïque, myristoleïque, palmitoleïque et linolénique.

2. Produit O-acylé dérivé du glucose susceptible d'être obtenu par estérification, partielle ou totale, de la vitamine F avec le glucose selon la revendication 1, et comprenant un mélange de composés de formule (1) suivante : 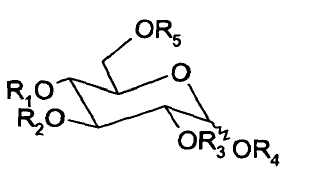 dans laquelle R1, R2, R3, R4 et R5, indépendamment les uns des autres, représentent l'hydrogène ou un radical -CO-R avec R représentant une chaîne hydrocarbonée linéaire, saturée ou insaturée, comprenant 11 à 21 atomes de carbone, sous réserve qu'au moins un des radicaux R1 à R5 soient différents de l'hydrogène.

3. Produit selon l'une des revendications précédentes, comprenant au moins un ester, notamment monoester, de glucose et d'acide linoléïque; au moins un ester, notamment monoester, de glucose et d'acide oléïque; au moins un ester, notamment monoester, de glucose et d'acide palmitique; et au moins un ester, notamment monoester, de glucose et d'acide stéarique.

4. Produit selon la revendication 3, comprenant en outre au moins un ester, notamment monoester, de glucose et d'un ou plusieurs esters de glucose et d'acide laurique, myristique, arachidique, béhénique, lauroleïque, myristoleïque, palmitoleïque et/ou linolénique.

5. Produit selon l'une des revendications 3 ou 4, comprenant en outre au moins un diester de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

6. Produit selon l'une des revendications précédentes comprenant, toutes positions confondues :
- de 40 à 80% en poids, de préférence 60 à 75% en poids, préférentiellement 68-72% en poids, d'ester de glucose et d'acide linoléïque,
- de 10 à 20% en poids, de préférence 12 à 17% en poids, préférentiellement 14-15% en poids, d'ester de glucose et d'acide oléique,
- de 5 à 20% en poids, de préférence 7 à 15% en poids, préférentiellement 9-12% en poids, d'ester de glucose et d'acide palmitique,
- de 0,5 à 7% en poids, de préférence 1 à 5% en poids, préférentiellement 2-4% en poids, d'ester de glucose et d'acide stéarique,
- de 0 à 10% en poids, notamment 0,10-4% en poids, voire 0,15-2% en poids, d'un ou plusieurs esters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléïque, palmitique, stéarique et/ou linolénique.

7. Produit selon l'une des revendications précédentes, dans lequel le glucose est estérifié en position 1, en position 2, en position 3 et/ou en position 6; préférentiellement, en position 1 et/ou 6.

8. Produit selon l'une des revendications précédentes comprenant :
- de 40 à 80% en poids, de préférence 60 à 75% en poids, préférentiellement 68-72% en poids, de monoester de glucose et d'acide linoléïque dont principalement le 6-0-octadeca-9,12-dienoyl-D-glucopyranose, le 1-O-octadeca-9,12-dienoyl-D-gluco-pyranose, le 2-O-octadeca-9,12-dienoyl-D-glucopyranose et/ou le 3-O-octadeca-9,12-dienoyl-D-glucopyranose,
- de 10 à 20% en poids, de préférence 12 à 17% en poids, préférentiellement 14-15% en poids, de monoester de glucose et d'acide oléique, dont principalement le 6-O-octadeca-9-enoyl-D-glucopyranose, le 3-O-octadeca-9-enoyl-D-glucopyranose, le 1-O-octadeca-9-enoyl-D-glucopyranose et/ou le 2-O-octadeca-9-enoyl-D-glucopy-ranose,
- de 5 à 20% en poids, de préférence 7 à 15% en poids, préférentiellement 9-12% en poids, de monoester de glucose et d'acide palmitique, dont principalement le 6-O-hexadecanoyl-D-glucopyranose, le 3-O-hexadecanoyl-D-glucopyranose, le 1-O-hexadecanoyl-D-glucopyranose et/ou le 2-O-hexadecanoyl-D-glucopyranose.
- de 0,5 à 7% en poids, de préférence 1 à 5% en poids, préférentiellement 2-4% en poids, de monoester de glucose et d'acide stéarique, dont principalement le 6-O-octadecanoyl-D-glucopyranose, le 3-O-octadecanoyl-D-glucopyranose, le 1-O-octadecanoyl-D-glucopyranose et/ou le 2-O-octadecanoyl-D-glucopyranose,
- de 0 à 10% en poids, notamment 0,10-4% en poids, voire 0,15-2% en poids, d'un ou plusieurs monoesters de glucose et d'acide laurique, myristique, arachidique, béhénique, lauroleïque, myristoleïque, palmitoleïque et/ou linolénique,
- 0 à 10% en poids, notamment 0,10-4% en poids, voire 0,15-2% en poids, de diesters de glucose et d'un ou plusieurs acides choisis parmi les acides laurique, myristique, arachidique, béhénique, lauroleïque, myristoleïque, palmitoleïque, linoléïque, oléique, palmitique, stéarique et/ou linolénique.

9. Produit selon l'une des revendications précédentes, dans lequel le rapport entre le nombre de fonctions esters du produit estérifié et le nombre de fonctions hydroxyles initiales, ou taux d'estérification, pour une molécule de glucose, est de préférence compris entre 0,2 et 1, notamment de 0,2 à 0,6 et en particulier de 0,21 à 0,4.

10. Produit selon l'une des revendications précédentes, dans lequel le rapport entre le nombre de fonctions esters en position 6 et le nombre total de fonctions esters, pour une molécule de glucose, est de préférence compris entre 0,55 et 1, notamment entre 0,70 et 0,98 et préférentiellement entre 0,90 et 0,97.

11. Composition comprenant, dans un milieu physiologiquement acceptable, au moins un produit selon l'une des revendications précédentes.

12. Composition selon la revendication 11, se présentant sous la forme d'une composition cosmétique ou pharmaceutique qui comprend un milieu cosmétiquement ou pharmaceutiquement acceptable.

13. Composition selon l'une des revendications 11 à 12, dans laquelle le produit est présent en une quantité comprise entre 0,01-20 % en poids par rapport au poids total de la composition, notamment entre 0,1-10% en poids, et de préférence entre 0,5-5% en poids.

14. Composition selon l'une des revendications 11 à 13, dans laquelle le milieu physiologiquement, cosmétiquement ou pharmaceutiquement acceptable est anhydre ou aqueux.

15. Composition selon l'une des revendications 11 à 14, dans laquelle le milieu comprend au moins un constituant choisi parmi l'eau, les solvants organiques, les tensioactifs, les émulsionnants, les épaississants ou gélifiants hydrophiles ou lipophiles, les agents cosmétiques, les conservateurs, les solvants, les antioxydants, les filtres UV, les agents alcalinisants ou acidifiants; les huiles et/ou les cires d'origine animale, minérale, végétale ou de synthèse; les charges, les matières colorantes telles que des pigments et/ou des colorants; les actifs cosmétiques ou pharmaceutiques.

16. Composition selon la revendication 15, dans laquelle les actifs cosmétiques ou pharmaceutiques sont choisis parmi, seuls ou en mélange :
- des composés améliorant l'activité sur la repousse et/ou sur le freinage de la chute des cheveux, comme les esters d'acide nicotinique, dont notamment le nicotinate de tocophérol, le nicotinate de benzyle et les nicotinates d'alkyles en C₁-C₆ comme les nicotinates de méthyle ou d'hexyle; les dérivés de pyrimidine, comme le 2,4-diamino 6-piperidinopyrimidine 3- oxyde; les dérivés de pyrimidine 3-oxyde;
- les agents antibactériens tels que les macrolides, les pyranosides et les tétracyclines, et notamment l'érythromycine ;
- les agents antagonistes de calcium, comme la cinnarizine, le diltiazem, la nimodipine et la nifedipine ;
- des hormones telles que l'estriol et ses analogues, la vitamine D et ses analogues, ou la thyroxine et ses sels ;
- des agents anti-inflammatoires stéroïdiens ou non stéroidiens, tels que les corticostéroïdes, par exemple l'hydrocortisone;
- des agents antiandrogènes, tels que l'oxendolone, la spironolactone, le diéthyls-tilbestrol et la flutamide ;
- des inhibiteurs stéroïdiens ou non stéroïdiens des 5-α-réductases tels que le finastéride ou l'acide 4,6-diméthoxy-indole 2-carboxylique ou ses dérivés;
- des agonistes potassiques tels que la cromakalim et le nicorandil.
- des agonistes des récepteurs de RXR des rétinoïdes et des antagonistes des rétinoïdes;
- des capteurs de radicaux OH, tels que le diméthylsulfoxyde ;
- des peptides comme par exemple le tripeptide Lys-Pro-Val, et plus généralement l'α-MSH et ses dérivés;
- des antipelliculaires ou antifongiques tels que le Zinc Pyrithione, la Piroctone Olamine, le disulfure de sélénium, la Tropolone, l'Hinokitiol, les complexes hinokitiol-zinc et hinokitiol-cuivre, les complexes métalliques divalents;
- des agents anti-radicaux libres; des antiséborrhéiques; des antiparasitaires; des antiviraux; des agents antiprurigineux.
- le diazoxyde, la spiroxasone, des phospholipides comme la lécithine, les acides linoléique et linolénique; l'acide salicylique et ses dérivés, comme les dérivés de l'acide salicylique porteurs d'un groupement alcanoyle ayant de 2 à 12 atomes de carbone en position 5 du cycle benzénique; des acides hydroxycarboxyliques ou cétocarboxyliques et leurs esters; des lactones et leurs sels correspondants; l'anthraline, des caroténoides; les acides eicosatétrayénoïque et eicosatriyénoïque ou leurs esters et amides; des extraits d'origine végétale ou bactérienne.
- les protéines ou les hydrolysats de protéine, les acides aminés, les polyols, l'urée, l'allantoïne, les sucres et les dérivés de sucre, les vitamines hydrosolubles, les extraits végétaux et les hydroxyacides;
- le rétinol (vitamine A) et ses dérivés, le tocophérol (vitamine E) et ses dérivés, les acides gras essentiels, les céramides, les huiles essentielles, l'acide salicylique et ses dérivés.

17. Composition selon l'une des revendications 11 à 16, se présentant sous forme de solution aqueuse ou huileuse; de dispersion du type lotion ou sérum; d'émulsion de type huile-dans-eau, eau-dans-huile ou multiple; de suspension ; de gel aqueux ou anhydre; de microcapsule ou microparticule; de dispersion vésiculaire de type ionique et/ou non ionique; de solution alcoolique ou hydroalcoolique; de crème, de gel, de mousse; de composition pour aérosol; de préparation solide tels que des savons ou des pains de nettoyage.

18. Composition selon l'une des revendications 11 à 17, se présentant sous forme d'une composition pour soins capillaires, et notamment un shampooing, une lotion ou une crème traitante, une lotion de mise en plis, une crème ou un gel coiffant; une composition de teinture, notamment teinture d'oxydation, éventuellement sous forme de shampooing colorant; une lotion restructurante pour les cheveux, une composition de permanente, notamment une composition pour le premier temps d'une permanente; une lotion ou un gel antichute, un shampooing antiparasitaire.

19. Procédé de traitement cosmétique des cheveux et/ou des poils, dans lequel on applique sur la peau, les cheveux et/ou les poils, une composition cosmétique selon l'une des revendications 12 à 18.

20. Procédé de traitement cosmétique des cheveux et/ou des poils, dans lequel on applique sur la peau, les cheveux et/ou les poils, une composition cosmétique comprenant une quantité efficace de produit selon l'une des revendications 1 à 10, à laisser celle-ci en contact avec la peau, les cheveux et/ou les poils, et éventuellement à rincer.

21. Procédé de traitement cosmétique selon l'une des revendications 19 à 20, pour améliorer l'esthétique des poils et/ou des cheveux, notamment en leur donnant une plus grande vigueur et/ou un aspect amélioré.

22. Procédé de traitement cosmétique selon l'une des revendications 19 à 21, pour améliorer l'état des poils et/ou des cheveux, et notamment pour diminuer et/ou freiner la chute des cheveux et/ou des poils, et/ou pour induire et/ou stimuler leur croissance.

23. Utilisation d'au moins un produit selon l'une des revendications 1 à 10, pour la préparation d'une composition, notamment pharmaceutique, destinée à améliorer l'état des poils et/ou des cheveux, et notamment destinée à diminuer et/ou freiner la chute des cheveux et/ou des poils, et/ou pour induire et/ou stimuler leur croissance.

24. Procédé de préparation de dérivés O-acylés majoritairement en position 6 du glucose de formule (la) : 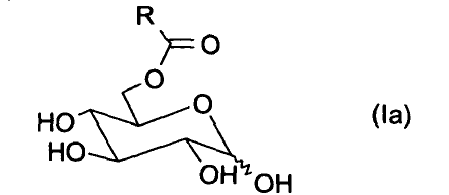 dans laquelle R est une chaîne hydrocarbonée linéaire ou ramifiée, saturée ou insaturée, comprenant 7 à 21 atomes de carbone, notamment 11 à 17 atomes de carbone,
comprenant les étapes consistant:
- dans une première étape, à préparer un anhydride mixte de formule (II) : 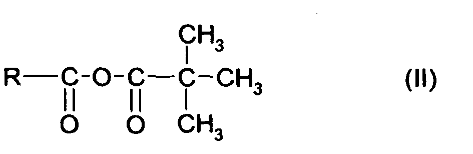 par réaction d'un acide carboxylique de formule R-COOH avec un halogénure d'acide triméthyle acétique de formule X-C(O)-C(CH₃)₃, avec X représentant de préférence le chlore ou le brome, et
- dans une seconde étape, à faire réagir ledit anhydride mixte formé avec le glucose.

25. Procédé selon la revendication 24, dans lequel le reste acyle -COR est un reste octanoyle, décanoyle, dodécanoyle, myristoyle, hexadécanoyle, stéaroyle, palmitoléoyle, oléoyle, linoléoyle ou linolénoyle, ou un mélange de ces restes.

26. Procédé selon l'une des revendications 24 à 25, dans lequel l'acide carboxylique est choisi parmi les acides octanoïque, décanoïque, dodécanoïque, myristique, hexadécanoïque, stéarique, oléique, linoléique ou linolénique, et leurs mélanges.

27. Procédé selon l'une des revendications 24 à 26, dans lequel la réaction de la première étape et/ou de la seconde étape est effectuée dans un solvant organique réactionnel, tel que le tétrahydrofuranne, le N,N-diméthylformamide, le N-méthylpyrrolidone, la pyridine, le toluène et leurs mélanges.

28. Procédé selon l'une des revendications 24 à 27, dans lequel la réaction de la première étape est effectuée en présence d'une base, notamment choisie parmi la triéthylamine, la pyridine, la 4-diméthylaminopyridine, la tributylamine, la N-méthylmorpholine et leurs mélanges.

29. Procédé selon l'une des revendications 24 à 28, dans lequel on fait réagir 0,3 à 3 équivalents, de préférence 0,5 à 1,5 équivalents, d'acide carboxylique avec 1 équivalent d'halogénure d'acide triméthyle acétique.

30. Procédé selon l'une des revendications 24 à 29, dans lequel on fait réagir 0,5 à 1,5, notamment 0,9 à 1,1, et encore mieux 1, équivalents d'anhydride mixte avec 3 équivalents de glucose.

31. Procédé selon l'une des revendications 24 à 30, dans lequel, dans le composé de formule (la), le rapport entre le nombre de fonctions esters en position 6 et le nombre total de fonctions esters, pour une molécule de glucose, est compris entre 55 et 95%, notamment entre 60 et 80%, préférentiellement de 68-75%.

32. Procédé selon l'une des revendications 24 à 31, permettant la préparation du 6-O-octadeca-9,12-dienoyl-D-glucopyranose; du 6-O-octadeca-9-enoyl-D-glucopyranose; du 6-O-octadecanoyl-D-glucopyranose; du 6-O-hexadecanoyl-D-glucopyranose; et de leurs mélanges.

33. Procédé selon l'une des revendications 24 à 31, permettant la préparation du produit tel que défini à l'une des revendications 1 à 10.

## Claims

1. O-Acyl product derived from glucose which may be obtained by partial or total esterification of vitamin F with glucose, said vitamin F being constituted of, in % by weight:
- from 75% to 80% by weight of linoleic acid,
- from 10% to 15% by weight of oleic acid,
- from 4% to 8% by weight of palmitic acid,
- from 0.5% to 3% by weight of stearic acid, and
- from 0 to 10% by weight of one or more other acids chosen from lauric acid, myristic acid, arachidic acid, behenic acid, lauroleic acid, myristoleic acid, palmitoleic acid and linolenic acid.

2. O-Acyl product derived from glucose which may be obtained by partial or total esterification of vitamin F with glucose according to Claim 1, and comprising a mixture of compounds of formula (I) below: 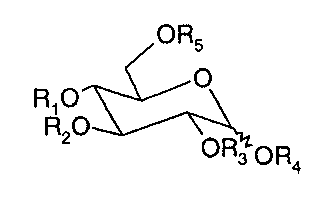 in which R1, R2, R3, R4 and R5, independently of each other, represent hydrogen or a radical -CO-R with R representing a saturated or unsaturated, linear hydrocarbon-based chain containing 11 to 21 carbon atoms, with the proviso that at least one of the radicals R1 to R5 is other than hydrogen.

3. Product according to either of the preceding claims, comprising at least one ester, especially a monoester, of glucose and of linoleic acid; at least one ester, especially a monoester, of glucose and of oleic acid; at least one ester, especially a monoester, of glucose and of palmitic acid; and at least one ester, especially a monoester, of glucose and of stearic acid.

4. Product according to Claim 3, also comprising at least one ester, especially a monoester, of glucose and of one or more esters of glucose and of lauric acid, myristic acid, arachidic acid, behenic acid, lauroleic acid, myristoleic acid, palmitoleic acid and/or linolenic acid.

5. Product according to either of Claims 3 and 4, also comprising at least one diester of glucose and of one or more acids chosen from lauric acid, myristic acid, arachidic acid, behenic acid, lauroleic acid, myristoleic acid, palmitoleic acid, linoleic acid, oleic acid, palmitic acid, stearic acid and/or linolenic acid.

6. Product according to one of the preceding claims, comprising, all positions considered together:
- from 40% to 80% by weight, preferably 60% to 75% by weight and preferentially 68-72% by weight of ester of glucose and of linoleic acid,
- from 10% to 20% by weight, preferably 12% to 17% by weight and preferentially 14-15% by weight of ester of glucose and of oleic acid,
- from 5% to 20% by weight, preferably 7% to 15% by weight and preferentially 9-12% by weight of ester of glucose and of palmitic acid,
- from 0.5% to 7% by weight, preferably 1% to 5% by weight and preferentially 2-4% by weight of ester of glucose and of stearic acid,
- from 0 to 10% by weight, especially 0.10-4% by weight, or even 0.15-2% by weight, of one or more esters of glucose and of lauric acid, myristic acid, arachidic acid, behenic acid, lauroleic acid, myristoleic acid, palmitoleic acid and/or linolenic acid,
- 0 to 10% by weight, especially 0.10-4% by weight, or even 0.15-2% by weight, of diesters of glucose and of one or more acids chosen from lauric acid, myristic acid, arachidic acid, behenic acid, lauroleic acid, myristoleic acid, palmitoleic acid, linoleic acid, oleic acid, palmitic acid, stearic acid and/or linolenic acid.

7. Product according to one of the preceding claims, in which the glucose is esterified in position 1, in position 2, in position 3 and/or in position 6; preferably in position 1 and/or 6.

8. Product according to one of the preceding claims, comprising:
- from 40% to 80% by weight, preferably 60% to 75% by weight and preferentially 68-72% by weight of monoester of glucose and of linoleic acid mainly comprising 6-O-octadeca-9,12-dienoyl-D-glucopyranose, 1-O-octadeca-9,12-dienoyl-D-glucopyranose, 2-O-octadeca-9,12-dienoyl-D-glucopyranose and/or 3-O-octadeca-9,12-dienoyl-D-glucopyranose,
- from 10% to 20% by weight, preferably 12% to 17% by weight and preferentially 14-15% by weight of monoester of glucose and of oleic acid mainly comprising 6-O-octadeca-9-enoyl-D-glucopyranose, 3-0-octadeca-9-enoyl-D-glucopyranose, 1-0-octadeca-9-enoyl-D-glucopyranose and/or 2-0-octadeca-9-enoyl-D-glucopyranose,
- from 5% to 20% by weight, preferably 7% to 15% by weight and preferentially 9-12% by weight of monoester of glucose and of palmitic acid mainly comprising 6-0-hexadecanoyl-D-glucopyranose, 3-O-hexadecanoyl-D-glucopyranose, 1-0-hexadecanoyl-D-glucopyranose and/or 2-O-hexadecanoyl-D-glucopyranose,
- from 0.5% to 7% by weight, preferably 1% to 5% by weight and preferentially 2-4% by weight of monoester of glucose and of stearic acid mainly comprising 6-O-octadecanoyl-D-glucopyranose, 3-0-octadecanoyl-D-glucopyranose, 1-0-octadecanoyl-D-glucopyranose and/or 2-O-octadecanoyl-D-glucopyranose,
- from 0 to 10% by weight, especially 0.10-4% by weight, or even 0.15-2% by weight, of one or more monoesters of glucose and of lauric acid, myristic acid, arachidic acid, behenic acid, lauroleic acid, myristoleic acid, palmitoleic acid and/or linolenic acid,
- 0 to 10% by weight, especially 0.10-4% by weight, or even 0.15-2% by weight, of diesters of glucose and of one or more acids chosen from lauric acid, myristic acid, arachidic acid, behenic acid, lauroleic acid, myristoleic acid, palmitoleic acid, linoleic acid, oleic acid, palmitic acid, stearic acid and/or linolenic acid.

9. Product according to one of the preceding claims, in which the ratio between the number of ester functions in the esterified product and the number of initial hydroxyl functions, or degree of esterification, per glucose molecule is preferably between 0.2 and 1, especially from 0.2 to 0.6 and in particular from 0.21 to 0.4.

10. Product according to one of the preceding claims, in which the ratio between the number of ester functions in position 6 and the total number of ester functions, per glucose molecule, is preferably between 0.55 and 1, especially between 0.70 and 0.98 and preferentially between 0.90 and 0.97.

11. Composition comprising, in a physiologically acceptable medium, at least one product according to one of the preceding claims.

12. Composition according to Claim 11, which is in the form of a cosmetic or pharmaceutical composition comprising a cosmetically or pharmaceutically acceptable medium.

13. Composition according to either of Claims 11 and 12, in which the product is present in an amount of between 0.01-20% by weight relative to the total weight of the composition, especially between 0.1-10% by weight and preferably between 0.5-5% by weight.

14. Composition according to one of Claims 11 to 13, in which the physiologically, cosmetically or pharmaceutically acceptable medium is anhydrous or aqueous.

15. Composition according to one of Claims 11 to 14, in which the medium comprises at least one constituent chosen from water, organic solvents, surfactants, emulsifiers, hydrophilic or lipophilic thickeners or gelling agents, cosmetic agents, preserving agents, solvents, antioxidants, UV-screening agents, acidifying or basifying agents; oils and/or waxes of animal, mineral, plant or synthetic origin; fillers, dyestuffs such as pigments and/or colorants; cosmetic or pharmaceutical active agents.

16. Composition according to Claim 15, in which the cosmetic or pharmaceutical active agents are chosen, alone or as a mixture, from:
- compounds for improving the activity with respect to regrowth of the hair and/or impeding hair loss, such as nicotinic acid esters, among which especially are tocopheryl nicotinate, benzyl nicotinate and C₁-C₆ alkyl nicotinates such as methyl or hexyl nicotinate; pyrimidine derivatives, for instance 2,4-diamino-6-piperidinopyrimidine 3-oxide; pyrimidine 3-oxide derivatives;
- antibacterial agents such as macrolides, pyranosides and tetracyclins, and especially erythromycin;
- calcium antagonists, for instance cinnarizine, diltiazem, nimodipine and nifedipine;
- hormones such as oestriol and its analogues, vitamin D and its analogues, or thyroxin and its salts;
- steroidal or non-steroidal antiinflammatory agents, such as corticosteroids, for example hydrocortisone;
- antiandrogens, such as oxendolone, spironolactone, diethylstilbestrol and flutamide;
- steroidal or non-steroidal inhibitors of 5-α-reductases, such as finasteride or 4,6-dimethoxyindole-2-carboxylic acid or its derivatives;
- potassium agonists such as cromakalim and nicorandil;
- retinoid RXR receptor agonists and retinoid antagonists;
- OH-radical scavengers such as dimethyl sulphoxide;
- peptides, such as, for example, the tripeptide Lys-Pro-Val, and more generally α-MSH and its derivatives;
- antidandruff or antifungal agents such as zinc pyrithione, piroctone olamine, selenium disulphide, tropolone, hinokitiol, hinokitiol-zinc and hinokitiol-copper complexes, divalent metal complexes;
- free-radical scavengers; anti-seborrhoeic agents; antiparasitic agents; antiviral agents; anti-pruriginous agents;
- diazoxide, spiroxazone, phospholipids, for instance lecithin, linoleic acid and linolenic acid; salicylic acid and its derivatives, for instance salicylic acid derivatives bearing an alkanoyl group containing from 2 to 12 carbon atoms in position 5 of the benzene ring; hydroxycarboxylic acids or keto carboxylic acids and esters thereof; lactones and the corresponding salts thereof; anthralin, carotenoids; eicosatetraenoic acid and eicosatrienoic acid or the esters and amides thereof; extracts of plant or bacterial origin;
- proteins or protein hydrolysates, amino acids, polyols, urea, allantoin, sugars and sugar derivatives, water-soluble vitamins, plant extracts and hydroxy acids;
- retinol (vitamin A) and its derivatives, tocopherol (vitamin E) and its derivatives, essential fatty acids, ceramides, essential oils, and salicylic acid and its derivatives.

17. Composition according to one of Claims 11 to 16, which is in the form of an aqueous or oily solution; a dispersion of the lotion or serum type; an emulsion of oil-in-water, water-in-oil or multiple type; a suspension; an aqueous or anhydrous gel; a microcapsule or microparticle; a vesicular dispersion of ionic and/or nonionic type; an alcoholic or aqueous-alcoholic solution; a cream, a gel or a mousse; an aerosol composition; a solid preparation such as cleansing soaps or bars.

18. Composition according to one of Claims 11 to 17, which is in the form of a haircare composition, and especially a shampoo, a medicated lotion or cream, a hair setting lotion, a styling cream or gel; a dye composition, especially an oxidation dye composition, optionally in the form of a colouring shampoo; a restructuring lotion for the hair, a permanent-waving composition, especially a composition for the first stage of a permanent-waving operation; a lotion or gel for preventing hair loss, or an antiparasitic shampoo.

19. Cosmetic process for treating head hair and/or other hairs, in which a cosmetic composition according to one of Claims 12 to 18 is applied to the skin, the head hair and/or other hairs.

20. Cosmetic process for treating head hair and/or other hairs, in which a cosmetic composition comprising an effective amount of product according to one of Claims 1 to 10 is applied to the skin, the head hair and/or other hairs, it is left in contact with the skin, the head hair and/or other hairs, and is optionally rinsed out.

21. Cosmetic treatment process according to either of Claims 19 and 20, for improving the aesthetic appearance of head hair and/or other hairs, especially by giving them greater vigour and/or improving their appearance.

22. Cosmetic treatment process according to one of Claims 19 to 21, for improving the condition of head hair and/or other hairs, and especially for reducing and/or impeding the loss of head hair and/or other hairs, and/or for inducing and/or stimulating their growth.

23. Use of at least one product according to one of Claims 1 to 10, for preparing a composition, especially a pharmaceutical composition, for improving the condition of head hair and/or other hairs, which is especially intended to reduce and/or impede the loss of head hair and/or other hairs, and/or to induce and/or stimulate their growth.

24. Process for preparing O-acyl derivatives mainly in position 6 of glucose, of formula (Ia): 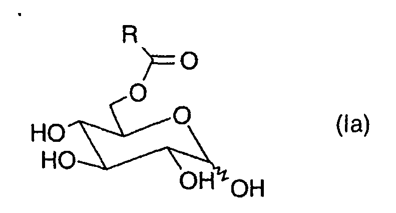 in which R is a linear or branched, saturated or unsaturated hydrocarbon-based chain containing 7 to 21 carbon atoms and especially 11 to 17 carbon atoms,
which comprises the steps consisting:
- in a first step, in preparing a mixed anhydride of formula (II): 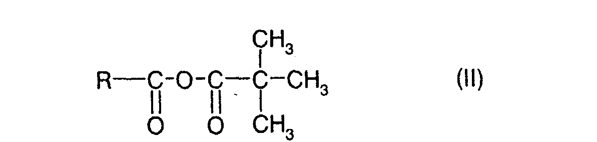 by reacting a carboxylic acid of formula R-COOH with a trimethylacetyl halide of formula
X-C (O) -C (CH₃) ₃, with X preferably representing chlorine or bromine, and
- in a second step, in reacting the said mixed anhydride formed with glucose.

25. Process according to Claim 24, in which the acyl residue -COR is an octanoyl, decanoyl, dodecanoyl, myristoyl, hexadecanoyl, stearoyl, palmitoleoyl, oleoyl, linoleoyl or linolenoyl residue, or a mixture of these residues.

26. Process according to either of Claims 24 and 25, in which the carboxylic acid is chosen from octanoic acid, decanoic acid, dodecanoic acid, myristic acid, hexadecanoic acid, stearic acid, oleic acid, linoleic acid and linolenic acid, and mixtures thereof.

27. Process according to one of Claims 24 to 26, in which the reaction of the first step and/or of the second step is performed in an organic reaction solvent such as tetrahydrofuran, N,N-dimethylformamide, N-methylpyrrolidone, pyridine or toluene, and mixtures thereof.

28. Process according to one of Claims 24 to 27, in which the reaction of the first step is performed in the presence of a base chosen especially from triethylamine, pyridine, 4-dimethylaminopyridine, tributylamine and N-methylmorpholine, and mixtures thereof.

29. Process according to one of Claims 24 to 28, in which 0.3 to 3 equivalents and preferably 0.5 to 1.5 equivalents of carboxylic acid are reacted with 1 equivalent of trimethylacetyl halide.

30. Process according to one of Claims 24 to 29, in which 0.5 to 1.5, especially 0.9 to 1.1 and better still 1, equivalent of mixed anhydride is reacted with 3 equivalents of glucose.

31. Process according to one of Claims 24 to 30, in which, in the compound of formula (Ia), the ratio between the number of ester functions in position 6 and the total number of ester functions, per glucose molecule, is between 55% and 95%, especially between 60% and 80% and preferably 68-75%.

32. Process according to one of Claims 24 to 31, for preparing 6-O-octadeca-9,12-dienoyl-D-glucopyranose; 6-0-octadeca-9-enoyl-D-glucopyranose; 6-O-octadecanoyl-D-glucopyranose; 6-0-hexadecanoyl-D-glucopyranose; and mixtures thereof.

33. Process according to one of Claims 24 to 31, for preparing the product as defined in one of Claims 1 to 10.

## Patentansprüche

1. O-acyliertes, von Glucose abgeleitetes Produkt, das durch vollständige oder partielle Veresterung von Vitamin F mit Glucose erhältlich ist, wobei das Vitamin F in Gewichtsprozent besteht aus:
- 75 bis 80 Gew.-% Linolsäure,
- 10 bis 15 Gew.-% Ölsäure,
- 4 bis 8 Gew.-% Palmitinsäure,
- 0,5 bis 3 Gew. -% Stearinsäure und
- 0 bis 10 Gew.-% einer oder mehrerer weiterer Säuren, die unter Laurinsäure, Myristinsäure, Arachinsäure, Behensäure, Lauroleinsäure, Myristoleinsäure, Palmitoleinsäure und Linolensäure ausgewählt sind.

2. O-acyliertes, von Glucose abgeleitetes Produkt, das durch vollständige oder partielle Veresterung von Vitamin F mit Glucose erhältlich ist, nach Anspruch 1, das ein Gemisch der Verbindungen der folgenden Formel (I) umfasst: 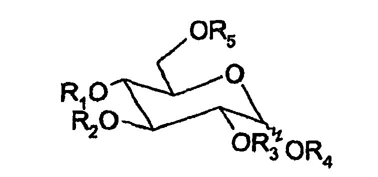 worin die Gruppen R₁, R₂, R₃, R₄ und R₅ unabhängig voneinander Wasserstoff oder eine Gruppe -CO-R bedeuten, wobei R eine geradkettige, gesättigte oder ungesättigte Kohlenwasserstoffkette mit 11 bis 21 Kohlenstoffatomen bedeutet, mit der Maßgabe, dass mindestens eine der Gruppen R₁ bis R₅ von Wasserstoff verschieden ist.

3. Produkt nach einem der vorhergehenden Ansprüche, das mindestens einen Ester und insbesondere einen Monoester von Glucose und Linolsäure; mindestens einen Ester und insbesondere einen Monoester von Glucose und Ölsäure; mindestens einen Ester und insbesondere einen Monoester von Glucose und Palmitinsäure; und mindestens einen Ester und insbesondere einen Monoester von Glucose und Stearinsäure umfasst.

4. Produkt nach Anspruch 3, das ferner mindestens einen Ester und insbesondere einen Monoester von Glucose und einem oder mehreren Estern von Glucose und Laurinsäure, Myristinsäure, Arachinsäure, Behensäure, Lauroleinsäure, Myristoleinsäure, Palmitoleinsäure und/oder Linolensäure umfasst.

5. Produkt nach einem der Ansprüche 3 oder 4, das ferner mindestens einen Diester von Glucose und einer oder mehreren Säuren umfasst, die unter Laurinsäure, Myristinsäure, Arachinsäure, Behensäure, Lauroleinsäure, Myristoleinsäure, Palmitoleinsäure, Linolsäure, Ölsäure, Palmitinsäure, Stearinsäure und/oder Linolensäure ausgewählt sind.

6. Produkt nach einem der vorhergehenden Ansprüche, das in allen Positionen zusammen umfasst:
- 40 bis 80 Gew.-%, vorzugsweise 60 bis 75 Gew.-% und bevorzugt 68 bis 72 Gew.-% Ester von Glucose und Linolsäure,
- 10 bis 20 Gew.-%, vorzugsweise 12 bis 17 Gew.-% und bevorzugt 14 bis 15 Gew.-% Ester von Glucose und Ölsäure,
- 5 bis 20 Gew.-%, vorzugsweise 7 bis 15 Gew.-% und bevorzugt 9 bis 12 Gew.-% Ester von Glucose und Palmitinsäure,
- 0,5 bis 7 Gew.-%, vorzugsweise 1 bis 5 Gew.-%, bevorzugt 2 bis 4 Gew.-% Ester von Glucose und Stearinsäure,
- 0 bis 10 Gew.-%, insbesondere 0,10 bis 4 Gew.-%, sogar 0,15 bis 2 Gew.-% eines oder mehrerer Ester von Glucose und Laurinsäure, Myristinsäure, Arachinsäure, Behensäure, Lauroleinsäure, Myristoleinsäure, Palmitoleinsäure und/oder Linolensäure,
- 0 bis 10 Gew.-%, insbesondere 0,10 bis 4 Gew.-%, sogar 0,15 bis 2 Gew.-% Diester von Glucose und einer oder mehreren Säuren, die unter Laurinsäure, Myristinsäure, Arachinsäure, Behensäure, Lauroleinsäure, Myristoleinsäure, Palmitoleinsäure, Linolsäure, Ölsäure, Palmitinsäure, Stearinsäure und/oder Linolensäure ausgewählt sind.

7. Produkt nach einem der vorhergehenden Ansprüche, wobei die Glucose in 1-Stellung, 2-Stellung, 3-Stellung und/oder 6-Stellung; bevorzugt in 1-Stellung und/oder 6-Stellung verestert ist.

8. Produkt nach einem der vorhergehenden Ansprüche, das umfasst:
- 40 bis 80 Gew.-%, vorzugsweise 60 bis 75 Gew.-%, bevorzugt 68 bis 72 Gew.-% Monoester von Glucose und Linolsäure, darunter hauptsächlich 6-O-Octadeca-9,12-dienoyl-D-glucopyranose, 1-O-Octadeca-9,12-dienoyl-D-glucopyranose, 2-O-Octadeca-9,12-dienoyl-D-glucopyranose und/oder 3-O-Octadeca-9,12-dienoyl-D-glucopyranose,
- 10 bis 20 Gew.-%, vorzugsweise 12 bis 17 Gew.-%, bevorzugt 14 bis 15 Gew.-% Monoester von Glucose und Ölsäure, darunter hauptsächlich 6-O-Octadeca-9-enoyl-D-glucose-pyranose, 3-O-Octadeca-9-enoyl-D-glucopyranose, 1-O-Octadeca-9-enoyl-D-glucopyranose und/oder 2-O-Octadeca-9-enoyl-D-glucopyranose,
- 5 bis 20 Gew.-%, vorzugsweise 7 bis 15 Gew.-%, bevorzugt 9 bis 12 Gew.-% Monoester von Glucose und Palmitinsäure, darunter hauptsächlich 6-O-Hexadecanoyl-D-glucopyranose, 3-O-Hexadecanoyl-D-glucopyranose, 1-O-Hexadecanoyl-D-glucopyranose und/oder 2-O-Hexadecanoyl-D-glycopyranose,
- 0,5 bis 7 Gew.-%, vorzugsweise 1 bis 5 Gew.-%, bevorzugt 2 bis 4 Gew.-% Monoester von Glucose und Stearinsäure, darunter hauptsächlich 6-O-Octadecanoyl-D-glucopyranose, 3-O-Octadecanoyl-D-glucopyranose, 1-O-Octadecanoyl-D-glucopyranose und/oder 2-O-Octadecanoyl-D-glucopyranose,
- 0 bis 10 Gew.-%, insbesondere 0,10 bis 4 Gew.-%, sogar 0,15 bis 2 Gew.-% eines oder mehrerer Monoester von Glucose und Laurinsäure, Myristinsäure, Arachinsäure, Behensäure, Lauroleinsäure, Myristoleinsäure, Palmitoleinsäure und/oder Linolensäure,
- 0 bis 10 Gew.-%, insbesondere 0,10 bis 4 Gew.-%, sogar 0,15 bis 2 Gew.-% Diester von Glucose und einer oder mehreren Säuren, die unter Laurinsäure, Myristinsäure, Arachinsäure, Behensäure, Lauroleinsäure, Myristoleinsäure, Palmitoleinsäure, Linolsäure, Ölsäure, Palmitinsäure, Stearinsäure und/oder Linolensäure ausgewählt sind.

9. Produkt nach einem der vorhergehenden Ansprüche, wobei das Verhältnis der Anzahl der Esterfunktionen des veresterten Produkts und der Anzahl der ursprünglichen Hydroxygruppen, oder der Veresterungsgrad, für ein Glucosemolekül vorzugsweise im Bereich von 0,2 bis 1, insbesondere 0,2 bis 0,6 und besonders 0,21 bis 0,4 liegt.

10. Produkt nach einem der vorhergehenden Ansprüche, wobei das Verhältnis der Anzahl der Esterfunktionen in 6-Stellung und der Anzahl der Esterfunktionen für ein Glucosemolekül vorzugsweise im Bereich von 0,55 bis 1, insbesondere 0,70 bis 0,98 und bevorzugt 0,90 bis 0,97 liegt.

11. Zusammensetzung, die in einem physiologisch akzeptablen Medium mindestens ein Produkt nach einem der vorhergehenden Ansprüche enthält.

12. Zusammensetzung nach Anspruch 11, die als kosmetische oder pharmazeutische Zusammensetzung vorliegt, welche ein kosmetisch oder pharmazeutisch akzeptables Medium enthält.

13. Zusammensetzung nach einem der Ansprüche 11 bis 12, wobei das Produkt in einem Mengenanteil von 0,01 bis 20 Gew.-%, bezogen auf das Gesamtgewicht der Zusammensetzung, insbesondere 0,1 bis 10 Gew.-% und bevorzugt 0,5 bis 5 Gew.-% enthalten ist.

14. Zusammensetzung nach einem der Ansprüche 11 bis 13, wobei das physiologisch, kosmetisch oder pharmazeutisch akzeptable Medium wasserfrei oder wässrig ist.

15. Zusammensetzung nach einem der Ansprüche 11 bis 14, wobei das Medium mindestens einen Bestandteil enthält, der unter Wasser, organischen Lösungsmitteln, grenzflächenaktiven Stoffen, Emulgatoren, hydrophilen oder lipophilen Verdickungsmitteln oder Gelbildnern, kosmetischen Stoffen, Konservierungsmitteln, Lösungsmitteln, Antioxidantien, UV-Filtern, Alkalisierungsmitteln oder Ansäuerungsmitteln; Ölen und/oder Wachsen tierischer, mineralischer, pflanzlicher oder synthetischer Herkunft; Füllstoffen, Farbmitteln, wie Pigmenten und/oder Farbstoffen; kosmetischen oder pharmazeutischen Wirkstoffen ausgewählt ist.

16. Zusammensetzung nach Anspruch 15, bei der die kosmetischen oder pharmazeutischen Wirkstoffe einzeln oder in Form von Gemischen ausgewählt sind unter:
- Verbindungen, die die Wirksamkeit bezüglich des Haarwuchses und/oder auf die Verlangsamung des Haarausfalls verbessern, wie Estern von Nicotinsäure, insbesondere Tocopherolnicotinat, Benzylnicotinat und C₁₋₆-Alkylnicotinaten, beispielsweise Methylnicotinat oder Hexylnicotinat; Pyrimidinderivaten, beispielsweise 2,4-Diamino-6-piperidino-pyrimidin-3-oxid; Derivaten von Pyrimidin-3-oxid;
- antibakteriellen Wirkstoffen, wie Makroliden, Pyranosiden und Tetracyclinen, insbesondere Erythromycin;
- Calcium-Antagonisten, wie Cinnarizin, Diltiazem, Nimodipin und Nifedipin;
- Hormonen, wie Estriol und seinen Analoga, Vitamin D und seinen Analoga oder Thyroxin und seinen Salzen;
- steroidalen oder nicht steroidalen entzündungshemmenden Wirkstoffen, wie Corticosteroiden, beispielsweise Hydrocortison;
- Antiandrogenen, wie Oxendolon, Spironolacton, Diethylstilbestrol und Flutamid;
- steroidalen oder nicht steroidalen Inhibitoren von 5-α-Reduktasen, wie Finasterid oder 4,6-Dimethoxyindol-2-carbonsäure und ihren Derivaten;
- Kalium-Agonisten, wie Cromakalim und Nicorandil;
- Agonisten von Retinoid-RXR-Rezeptoren und Antagonisten von Retinoiden;
- OH-Radikalfängern, wie Dimethylsulfoxid;
- Peptiden, wie beispielsweise dem Tripeptid Lys-Pro-Val und allgemeiner α-MSH und seinen Derivaten;
- Antischuppenmitteln oder antimykotischen Wirkstoffen, wie Zink-Pyrithion, Piroctone Olamine, Selendisulfid, Tropolon, Hinokitiol, Hinokitiol-Zink-Komplexen und Hinokitiol-Kupfer-Komplexen, Komplexen von zweiwertigen Metallen;
- Radikalfängern für freie Radikale; Antiseborrhoika; antiparasitären Wirkstoffen; antiviralen Wirkstoffen; juckreizstillenden Wirkstoffen;
- Diazoxid, Spiroxason, Phospholipiden, wie Lecithin, Linolsäure und Linolensäure; Salicylsäure und ihren Derivaten, beispielsweise Salicylsäurederivaten, die in 5-Stellung des Benzolrings eine Alkanoylgruppe mit 2 bis 12 Kohlenstoffatomen aufweisen; Hydroxycarbonsäuren oder Ketocarbonsäuren und ihren Estern; Lactonen und ihren entsprechenden Salzen; Anthralin, Carotinoiden; Eicosatetraensäure und Eicosatriensäure oder deren Estern und Amiden; Extrakten pflanzlicher Herkunft oder bakterieller Herkunft;
- Proteinen oder Proteinhydrolysaten, Aminosäuren, Polyolen, Harnstoff, Allantoin, Zuckern und Zuckerderivaten, wasserlöslichen Vitaminen, Pflanzenextrakten und Hydroxysäuren;
- Retinol (Vitamin A) und seinen Derivaten, Tocopherol (Vitamin E) und seinen Derivaten, essentiellen Fettsäuren, Ceramiden, etherischen Ölen, Salicylsäure und ihren Derivaten.

17. Zusammensetzung nach einem der Ansprüche 11 bis 16, die als wässrige oder ölige Lösung; Dispersion vom Typ Lotion oder Serum; Emulsion vom Öl-in-Wasser-Typ, Wasser-in-Öl-Typ oder multiplen Typ; Suspension; wässriges oder wasserfreies Gel; Mikrokapsel oder Mikropartikel; Vesikeldispersion vom ionischen und/oder nichtionischen Typ; alkoholische oder wässrigalkoholische Lösung; Creme, Gel, Schaum; Zusammensetzung für Aerosole; festes Präparat wie Seifen oder Reinigungsstücke vorliegt.

18. Zusammensetzung nach einem der Ansprüche 11 bis 17, die als Zusammensetzung für die Pflege der Haare und insbesondere als Haarwaschmittel, Lotion oder Creme für die Behandlung, Lotion für Wasserwellen, Frisiercreme oder Frisiergel; Zusammensetzung zum Färben und insbesondere zum oxidativen Färben, gegebenenfalls in Form eines färbenden Haarwaschmittels; restrukturierende Lotion für die Haare, Zusammensetzung für dauerhafte Verformungen und insbesondere Zusammensetzung für den ersten Schritt einer Dauerwelle; Lotion oder Gel gegen Haarausfall, antiparasitäres Haarwaschmittel vorliegt.

19. Verfahren zur kosmetischen Behandlung der Haare und/oder der Körperhaare, bei dem auf die Haut, die Haare und/oder die Körperhaare eine kosmetische Zusammensetzung nach einem der Ansprüche 12 bis 18 aufgebracht wird.

20. Verfahren zur kosmetischen Behandlung der Haare und/oder der Körperhaare, bei dem auf die Haut, die Haare und/oder die Körperhaare eine kosmetische Zusammensetzung aufgebracht wird, die eine wirksame Menge des Produkts nach einem der Ansprüche 1 bis 10 enthält, diese mit der Haut, den Haaren und/oder den Körperhaaren in Kontakt belassen und gegebenenfalls gespült wird.

21. Verfahren zur kosmetischen Behandlung nach einem der Ansprüche 19 bis 20, um das ästhetische Aussehen der Körperhaare und/oder der Haare zu verbessern, insbesondere um ihnen eine größere Spannkraft und ein verbessertes Aussehen zu geben.

22. Verfahren zur kosmetischen Verhandlung nach einem der Ansprüche 19 bis 21, um den Zustand der Körperhaare und/oder der Haare zu verbessern und insbesondere um das Ausfallen der Haare und/oder Körperhaare zu vermindern und/oder zu verlangsamen und/oder das Haarwachstum zu induzieren und/oder zu stimulieren.

23. Verwendung mindestens eines Produkts nach einem der Ansprüche 1 bis 10 für die Herstellung einer Zusammensetzung und insbesondere einer pharmazeutischen Zusammensetzung, die dazu vorgesehen ist, den Zustand der Körperhaare und/oder der Haare zu verbessern und insbesondere das Ausfallen der Haare und/oder der Körperhaare zu vermindern und/oder zu verlangsamen und/oder das Haarwachstum zu induzieren und/ oder zu stimulieren.

24. Verfahren zur Herstellung von hauptsächlich in 6-Stellung O-acylierten Glucosederivaten der Formel (la): 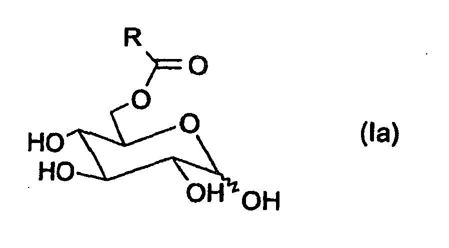 worin R eine geradkettige oder verzweigte, gesättigte oder ungesättigte Kohlenwasserstoffkette mit 7 bis 21 Kohlenstoffatomen und insbesondere 11 bis 17 Kohlenstoffatomen ist, das die folgenden Schritte umfasst:
- in einem ersten Schritt wird ein gemischtes Anhydrid der folgenden Formel (II): 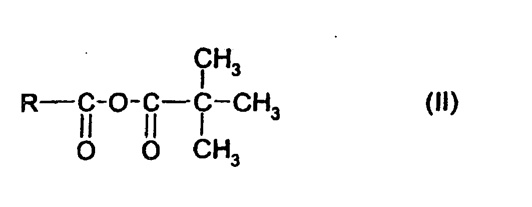 durch Umsetzung einer Carbonsäure der Formel R-COOH mit einem Halogenid von Trimethylessigsäure der Formel X-C(O)-C(CH₃)₃ hergestellt, wobei X vorzugsweise Chlor oder Brom bedeutet, und
- in einem zweiten Schritt wird das gebildete gemischte Anhydrid mit Glucose umgesetzt.

25. Verfahren nach Anspruch 24, wobei es sich bei dem Acylrest -COR um Octanoyl, Decanoyl, Dodecanoyl, Myristoyl, Hexadecanoyl, Stearoyl, Palmitoleoyl, Oleoyl, Linoleoyl oder Linolenoyl oder ein Gemisch dieser Reste handelt.

26. Verfahren nach einem der Ansprüche 24 bis 25, wobei die Carbonsäure unter Octansäure, Decansäure, Dodecansäure, Myristinsäure, Hexadecansäure, Stearinsäure, Ölsäure, Linolsäure oder Linolensäure oder deren Gemischen ausgewählt ist.

27. Verfahren nach einem der Ansprüche 24 bis 26, wobei die Umsetzung des ersten Schrittes und/oder des zweiten Schrittes in einem organischen Lösungsmittel durchgeführt wird, wie Tetrahydrofuran, N,N-Dimethylformamid, N-Methylpyrrolidon, Pyridin, Toluol und deren Gemischen.

28. Verfahren nach einem der Ansprüche 24 bis 27, wobei die Umsetzung des ersten Schrittes in Gegenwart einer Base durchgeführt wird, die insbesondere unter Triethylamin, Pyridin, 4-Dimethylaminopyridin, Tributylamin, N-Methylmorpholin und deren Gemischen ausgewählt ist.

29. Verfahren nach einem der Ansprüche 24 bis 28, bei dem 0,3 bis 3 Äquivalente und vorzugsweise 0,5 bis 1,5 Äquivalente Carbonsäure mit einem Äquivalent Trimethylessigsäurehalogenid umgesetzt werden.

30. Verfahren nach einem der Ansprüche 24 bis 29, bei dem 0,5 bis 1,5 und insbesondere 0,9 bis 1,1 Äquivalente und besser 1 Äquivalent gemischtes Anhydrid mit 3 Äquivalenten Glucose umgesetzt werden.

31. Verfahren nach einem der Ansprüche 24 bis 30, bei dem das Verhältnis zwischen der Anzahl der Esterfunktionen in 6-Stellung und der Gesamtzahl der Esterfunktionen für ein Glucosemolekül in der Verbindung der Formel (Ia) im Bereich von 55 bis 95 %, insbesondere 60 bis 80 % und bevorzugt 68 bis 75 % liegt.

32. Verfahren nach einem der Ansprüche 24 bis 31, nach dem 6-O-Octadeca-9,12-dienoyl-D-glucopyranose; 6-O-Octodeca-9-enoyl-D-glucopyranose; 6-O-Octadecanoyl-D-glucopyranose; 6-O-Hexadecanoyl-D-glucopyranose; und deren Gemische hergestellt werden können.

33. Verfahren nach einem der Ansprüche 24 bis 31, das die Herstellung des Produkts nach einem der Ansprüche 1 bis 10 ermöglicht.
